# EUROPEAN PATENT APPLICATION

(11) **EP 4 245 302 A1**
(43) Date of publication of application: **20.09.2023**
(21) Application number: 22162072.7
(22) Date of filing: 15.03.2022
(51) Int. Cl.: A61K 31/404, A61K 31/443, A61K 31/47, A61P 7/02, A61K 31/4439

(54) **CFTR MODULATORS FOR THE TREATMENT OF VASCULAR DISEASE**

(71) Applicant: Charité - Universitätsmedizin Berlin, 10117 Berlin (DE)
(72) Inventor: Kübler, Wolfgang, Berlin (DE); Simmons, Szandor, Berlin (DE); Asmus, Erik, Berlin (DE); Szulcek, Robert, Berlin (DE); Preißner, Robert, Berlin (DE)
(74) Representative: Hertin und Partner Rechts- und Patentanwälte PartG mbB

(57) **Abstract**

The invention relates to the field of pharmaceutical compositions and the treatment and/or prevention of medical conditions comprising thrombosis, or atherosclerosis associated with pathological thrombocyte activation. The invention further relates to a cystic fibrosis transmembrane conductance regulator (CFTR) modulator for use in the treatment and/or prevention of a medical condition comprising thrombosis, or a medical condition comprising atherosclerosis and pathological thrombocyte activation, in a human subject. The invention further relates to the medical use and corresponding therapeutic methods of administering a CFTR modulator, such as ivacaftor, in the treatment and/or prevention of a medical condition pathological thrombocyte activation, adhesion and/or aggregation. In further aspects, the invention relates to a pharmaceutical composition comprising a CFTR modulator, such as ivacaftor.

## Description

The invention relates to the field of pharmaceutical compositions and the treatment and/or prevention of medical conditions comprising thrombosis, or atherosclerosis associated with pathological thrombocyte activation.

The invention further relates to a cystic fibrosis transmembrane conductance regulator (CFTR) modulator for use in the treatment and/or prevention of a medical condition comprising thrombosis in a human subject.

The invention further relates to a cystic fibrosis transmembrane conductance regulator (CFTR) modulator for use in the treatment and/or prevention of a medical condition comprising atherosclerosis associated with pathological thrombocyte activation in a human subject.

The invention further relates to the medical use and corresponding therapeutic methods of administering a CFTR modulator, such as ivacaftor, in the treatment and/or prevention of a medical condition pathological thrombocyte activation, adhesion and/or aggregation.

In further aspects, the invention relates to a pharmaceutical composition comprising a CFTR modulator, such as ivacaftor.

### BACKGROUND OF THE INVENTION

Blood clotting is an essential protective mechanism of the body and requires the activation of thrombocytes. Blood clot or blood plug (thrombus) formed in the blood vessels are associated with many serious and even life-threatening diseases, such as thrombosis, thromboembolism, pneumonia, infectious diseases, inflammatory diseases, rheumatic diseases, hematological diseases (e.g., thrombotic thrombocytopenic purpura), and cardiovascular diseases,(e.g., atherosclerosis, stroke, myocardial infarction).

Activation of thrombocytes causes release of a number of pro-inflammatory substances, such as eicosanoids, interleukins and chemokines. At high local concentrations of these substances, genes of the so-called early inflammatory response are expressed in the actually anti-thrombotic endothelial cells of the vessels, which may alter the chemotactic and adhesive properties of the endothelium locally and systemically. These changes may act as drivers of many disease processes, even COVID-19. As a consequence, interactions between platelets and endothelial cells are enhanced and thrombus formation is initiated. A combination of disseminated intravascular coagulation and thrombotic microangiopathy then frequently leads to fatal thromboembolic events. In addition, after surviving pneumonia, the rate of cardiovascular events, such as myocardial infarction and stroke, remains elevated for many years.

Thrombosis is a vascular disease or disorder of the circulatory system in which a blood clot (thrombus, blood plug) forms (intravitally) in a blood vessel. Thrombosis can occur in any vessel. Most often, it is a thrombosis of the veins, specifically a thrombosis of the deep veins of the leg. Vascular diseases affect arteries and veins of the circulatory system causing a variety of health problems, such as hypertension, stroke, aneurysms, and peripheral artery disease, wherein many of these are serious or even lethal.

Pulmonary vascular micro- and macrothrombosis has been observed in 20-30% of patients with COVID-19; a proportion significantly higher than in other critically ill patient groups (1-10%).¹⁻⁷ The pulmonary or systemic pro-inflammatory processes induced by such bacterial or viral pneumonia or by resulting systemic sepsis result in, among other things, systemic activation of blood coagulation (hemostasis). The resulting tendency to coagulopathy is considered a key risk factor for the high morbidity and mortality of community acquired or hospital-acquired pneumonia (CAP or HAP, respectively) or COVID-19 disease. ^{8,9}

In addition to the disease complex of CAP, HAP and COVID-19, thromboembolic conditions are an important factor in numerous local and systemic diseases, which, however, have so far been insufficiently addressed therapeutically or preventively, for example systemic or extrapulmonary infectious and inflammatory diseases, hematological diseases, and cardiovascular diseases. These diseases have a high individual, economic and socioeconomic burden.

Many patients suffer from a form of thrombosis, coagulopathies, and/or thromboembolic events and associated heath consequences for decades with no prospect of a cure and often with fatal outcome. Health, individual, economic and socioeconomic burden are high. Prophylaxis and therapy of coagulopathies are still mostly empirical. The need for effective and approved pharmacological therapy for the treatment and prevention of thrombosis, coagulopathies and thromboembolic events is urgent. Numerous clinical trials of pharmacologic interventions that addressed this problem failed.¹⁰⁻¹²

Despite the high medical need, efficacious treatments remain limited and additional treatment options are urgently needed.

### SUMMARY OF THE INVENTION

In light of the prior art, the technical problem underlying the present invention is to provide alternative or improved means for the treatment and/or prevention of a medical condition comprising thrombosis. The technical problem may also be viewed as the provision of means for the treatment and/or prevention of a medical condition comprising a pathological thrombocyte activation, particularly a thrombocyte hyperactivation.

The technical problem may also be viewed as the provision of means for the treatment and/or prevention of a medical condition comprising atherosclerosis and thrombocyte hyperactivation. The technical problem may also be viewed as the provision of means for treating and/or preventing of medical conditions comprising a vascular disease associated with a hypercoagulative, a thrombotic- and/or a thromboembolic event.

The technical problem may also be viewed as the provision of means for treating and/or preventing of developing a thrombocytopathy, a coagulopathy and/or an embolism, particularly occurring with or persisting after onset of an infection, such as COVID-19.

This problem is solved by the features of the independent claims. Preferred embodiments of the present invention are provided by the dependent claims.

In one aspect, the invention relates to a cystic fibrosis transmembrane conductance regulator (CFTR) modulator for use in the treatment and/or prevention of a medical condition comprising thrombosis in a human subject.

As described at length herein, the use of a CFTR modulator for treating and/or preventing pathological thrombocyte activation, aggregation, and/or adhesion, atherosclerosis, thrombosis, thromboembolism, represents a novel and advantageous approach towards developing an effective therapeutic measure for diseases and disorders described herein.

At present, there is no effective medication available. Experiments with thrombocytes of patients, e.g., COVID-19 blood cells, are being conducted, providing proof of CFTR modulators being effective in treating the medical conditions described herein, particularly pathological thrombocyte activation, aggregation, and/or adhesion.

In one embodiment, the CFTR modulator is administered to a human subject to treat thrombosis.

Under non-pathological physiological conditions, in primary hemostasis, thrombocytes are activated by the process of thrombocyte adhesion and further inducing coagulation and thrombocyte aggregation for the formation of a white thrombus. These mechanisms promote activation and aggregation of additional thrombocytes, for secondary hemostasis, comprising activation of thrombocytes and formation of active thrombin, and coagulation. Therefore, thrombocyte dysfunction may be characterized by pathological aggregation, proliferation, activation, or adhesion of thrombocytes.

In some embodiments, a pathological thrombocyte activation comprises a thrombocyte hyperactivation.

To the knowledge of the inventors, it has not been previously disclosed or suggested, that modulation of CFTR function changes thrombocyte function from hyperactivated, which is pathological, into a physiological activated (non-pathological) condition.

In one embodiment, the pathological thrombocyte activation is also a thrombocyte dysfunction. In one embodiment, the pathological thrombocyte activation comprises thrombocyte hyperactivation, pathological thrombocyte aggregation, pathological thrombocyte adhesion, and/or pathological thrombocyte-mediated coagulation, such as hypercoagulability.

In some embodiments, the CFTR modulator is used to treat and/or to prevent a pathological thrombocyte activation, preferably thrombocyte hyperactivation.

In another embodiment, the CFTR modulator is used to treat and/or to prevent medical conditions associated with pathological thrombocyte activation, preferably thrombocyte hyperactivation.

In one embodiment, the pathological thrombocyte activation is associated with embolism and/or a thrombocyte-mediated coagulopathy, such as a hypercoagulability/thrombophilia.

In one embodiment, the CFTR modulator is used to treat thrombocytopathy, coagulopathy, and/or embolism. In one embodiment, the CFTR modulator is used to prevent thrombocytopathy, coagulopathy, and/or embolism.

As shown in Example 1, the inventors identified the cystic fibrosis transmembrane conductance regulator (CFTR), a chloride channel, as an important regulator for normalizing thrombocyte function in COVID-19 patients. The inventors demonstrate that treatment with CFTR modulator ivacaftor attenuates agonist-induced thrombocyte activation, aggregation and adhesion. These protective properties were preserved in thrombocytes of COVID-19 patients, pointing towards a therapeutic potential of CFTR- modulators in the therapy of e.g. severe COVID-19.

In one embodiment, administration of a CFTR modulator treats and/or prevents a medical condition comprising a thrombocyte dysfunction.

In some embodiments, the subject is or has been treated additionally with a drug, such as a drug approved by FDA or EMA, for the treatment of a medical condition as described herein. In some embodiments, the additional treatment is not or does not comprise a CFTR modulator. In some embodiments, the invention relates to the combined administration of a therapeutically effective amount of a CFTR modulator, and a non-CFTR modulator treatment.

In one embodiment, symptoms of a medical condition described herein may be acute and/or chronic. In one embodiment, symptoms of a medical condition described herein may be new, recurrent, intermittent, episodic, transitory, or persistent.

In one embodiment, the effectiveness and physiological response of the treatment, as described herein, is determined by measuring activation marker expressed on the surface of thrombocytes (e.g.,CD62p, CD63, CD41/CD61), measuring calcium influx in thrombocytes, or thrombosis diagnostics (for example, using Wells score for leg vein thrombosis, compression ultrasound, computed tomography, or magnetic resonance imaging).

Patients suffering from diseases described herein suffer from a significantly higher risk for death. A particular advantage of a treatment using CFTR modulators is the reduction of the risk of death, if not completely eliminated.

In some embodiments, the medical condition comprises an embolism and/or a thrombocyte-mediated coagulopathy, such as a hypercoagulability/thrombophilia.

In the prior art, treatment with CFTR modulators is not known to result in normalization of thrombocyte function or being effective for coagulopathy, to change thrombocytes function from a hypercoagulative to a normal-coagulative state. In one embodiment, the CFTR modulator prevents thrombocyte hyperactivation and/or hypercoagulability.

Surprisingly, the CFTR modulator exhibit anti-coagulant effects via an increase in the activity of CFTR, opposing the development of thrombocytopathy, coagulopathy, and/or embolism in, for example, bacterial and viral pneumonias.

As shown in Example 1, thrombocytes of COVID-19 patients are hyperactivated as found by over expression of activation markers at the surface (CD62p, CD63, CD41/CD61) and increase calcium influx. Thrombocytes from COVID-19 patients showed enhanced surface expression of the activation markers CD62p (p-selectin) and CD63 as a function of disease severity, demonstrating a hypercoagulable state in COVID- 19. Remarkably, increased expression of these activation markers could be recapitulated in platelets of healthy donors stimulated with either of the three thrombocyte agonists adenosine diphosphate (ADP), thrombin receptor activating protein-6 (TRAP6), or platelet activating factor (PAF).

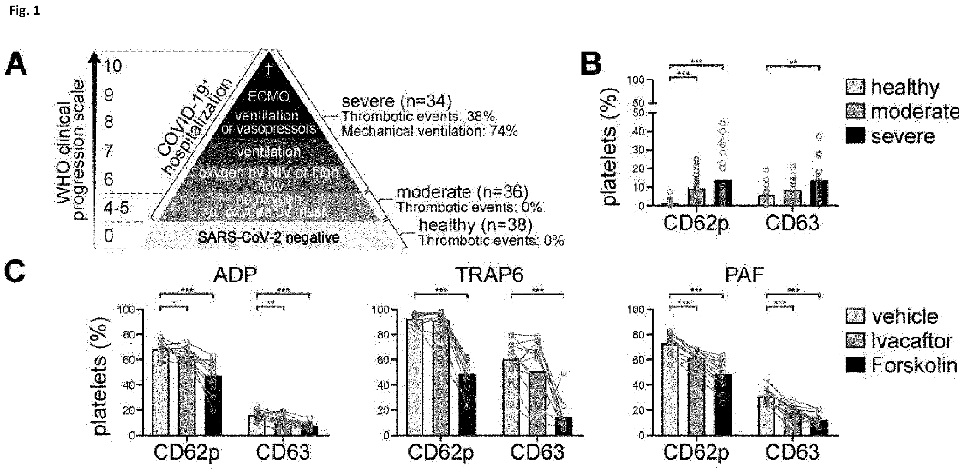

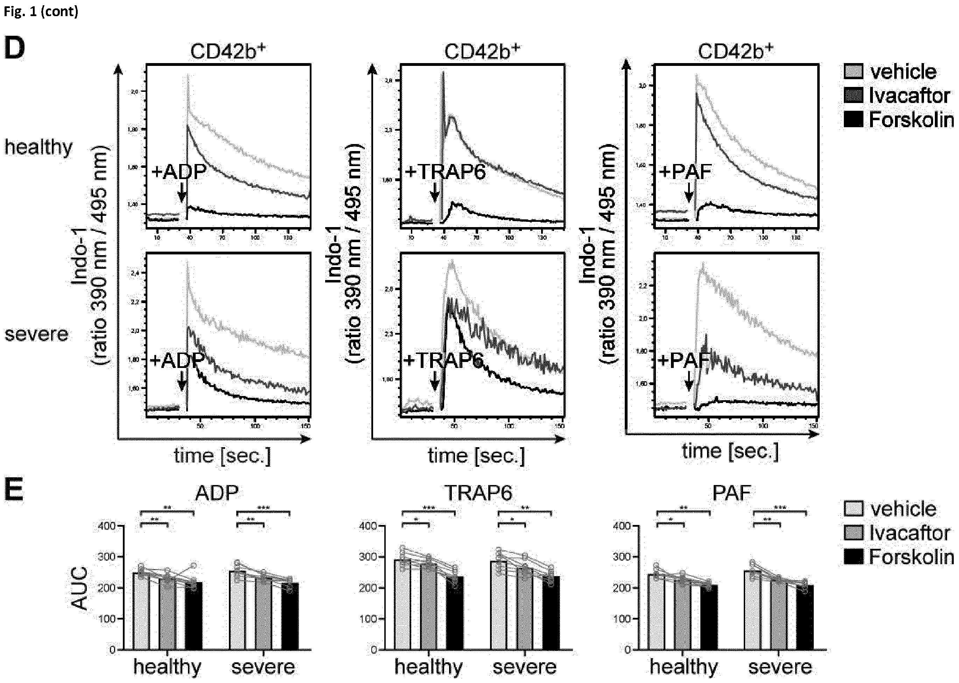

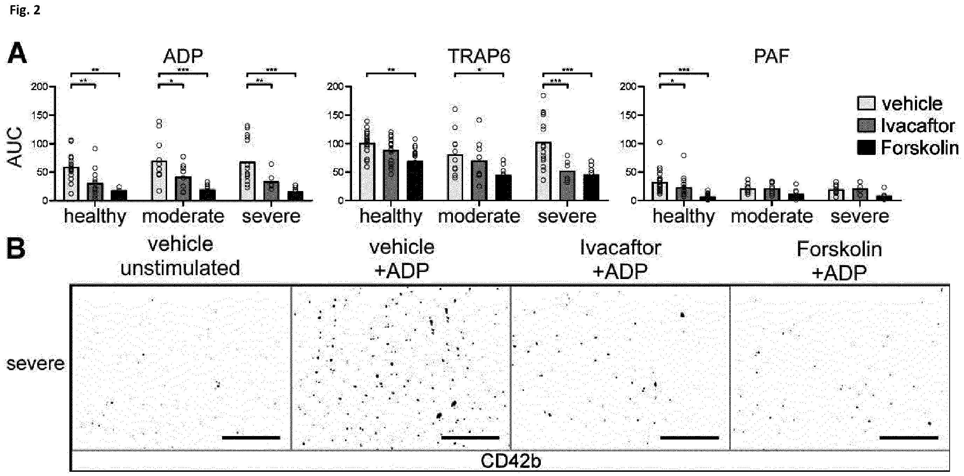

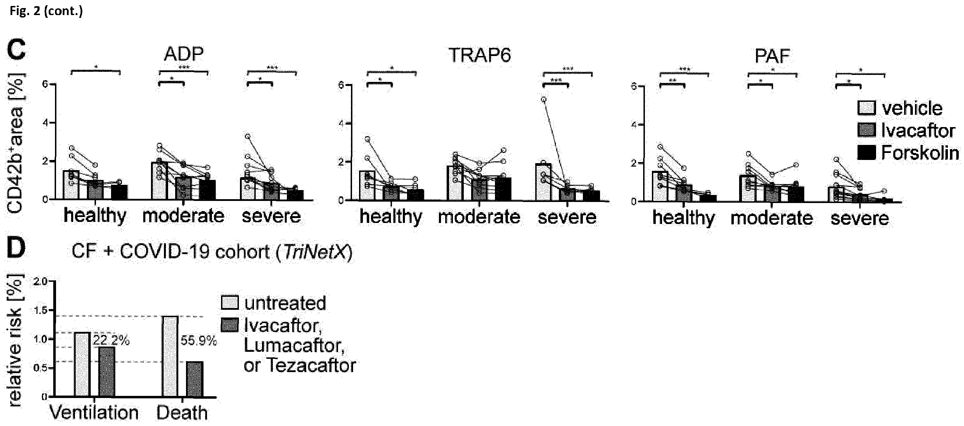

These findings are relevant for pneumonia-associated hyperactivation of thrombocytes in COVID-19. Agonist-induced thrombocyte hyperactivation was normalized by treatment and pre-treatment with CFTR modulator ivacaftor to physiological thrombocyte activation (Figure 1, 2). The inventors show that pre-treatment of thrombocytes using the CFTR-potentiator ivacaftor leads to a significant reduction in calcium influx through calcium channels. For reference purposes, the inventors used in their experiments forskolin in parallel, an activator of adenylate cyclase commonly used in experimental studies to activate CFTR. Surprisingly, the protective effects of ivacaftor qualitatively resembled those of forskolin. The non-specific effects of generalized adenylate cyclase activation prohibit clinical use of forskolin, wherein CFTR-modulators have proven safe and effective in the treatment of CF patients and their use, particularly in long term, has not been associated with a higher risk of bleeding disorders.

In one embodiment, the CFTR modulator is used to treat hypercoagulability/thrombophilia.

In some embodiments, the CFTR modulator is administered to a subject who has, is suspected to have or is at risk of having thrombocyte-mediated coagulopathy, such as hypercoagulability/thrombophilia.

In one embodiment, CFTR modulator treatment reduces a thrombocyte-mediated coagulopathy. In some embodiments, CFTR modulator improve one or more symptoms of coagulopathy. The CFTR modulator for use in an interventional therapy as described herein is intended to prevent, to encompass a slowing or reduction in disease progression comprising thrombocyte-mediated coagulopathy, such as a hypercoagulability/thrombophilia or a reduction of an acute or non-acute embolism. In some embodiments, CFTR modulator improve one or more symptoms of embolism.

In one embodiment, the effectiveness and physiological response of the treatment, as described herein, is determined by measuring thrombocyte-mediated coagulopathy, for example as described in Example 1.

As typically understood in the art, calcium is the main "second messenger" of thromobocyte activation. The inventors have shown a significant effect of ivacaftor on agonist-induced increases in intracellular calcium levels. Activation with either ADP, TRAP6, or PAF induced a well-defined [Ca2+]i response in thrombocytes of healthy donors or severe COVID-19 patients. Surprisingly, pretreatment of thrombocytes with ivacaftor significantly attenuated the agonist-induced calcium increase (Example 1).

In some embodiments, the subject of treatment has suffered a thrombotic- and/or thromboembolic event.

Thrombotic and/or thromboembolic events play an important role in numerous local and systemic diseases. A pulmonary vascular micro- and macrothrombosis particulary occur in critical ill patients and is associated with significant morbidity and lethality for these patients In one embodiment, CFTR modulators for the prevention and/or treatment of pulmonary disorders. In one embodiment, treatment using CFTR modulators reduces lethality in pulmonary disorders. Of particular advantage is the reduction of the risk of death by treatment with a CFTR modulator, if not completely absent.

In some embodiments, the CFTR modulator is administered to a subject who has, is suspected to have or is at risk of developing a thrombotic event. In one embodiment, the thrombotic event is a venous thrombosis, such as a superficial venous thromboses, a deep vein thrombosis, a pulmonary embolism, a lung embolism, a superficial thrombophlebitis, or a venous thromboembolism. In one embodiment, the thrombotic event is an arterial thrombosis, such as a myocardial infarction, an ischemic stroke, a critical limb ischaemia, a stroke or a mesenteric ischemia. In one embodiment, the thrombotic event occurs in a cardiac chamber. In one embodiment, a thrombus blocks blood flow at the site of its formation. In one embodiment, the thrombotic event comprises developing one or more thrombus. In one embodiment, a thromboembolic event comprise thrombus dislodged by the bloodstream, and wherein said thrombus embolize distant vessels.

In some embodiments, the CFTR modulator is administered to a subject who has, is suspected to have or is at risk developing a thromboembolic event, such as a venous thromboembolism.

In embodiments, thrombosis is a gradual process that can lead to blockage and loss of blood vessels and/or vascular systems. In one embodiment, CFTR modulator is administered to subjects who is suspected to have or is at risk of developing a thrombotic and/or thromboembolic event. The treatment using CFTR modulator can prevent blockage and loss of blood vessels and/or vascular systems. A particular benefit of treatment using CFTR modulator is the significant reduction in suffering a thrombotic and/or thromboembolic event or dying from it. CFTR modulators are known to be well tolerated. The treatment can also be given over a long period of time, even years, without causing severe side effects. This significantly reduces the mortality rate associated with a thrombotic and/or thromboembolic event.

In one embodiment, CFTR modulator treatment reduces developing a thrombotic and/or thromboembolic event. The CFTR modulator for use in an interventional therapy as described herein is intended to prevent, to encompass a slowing or to reduce progression a thrombotic and/or thromboembolic event, such thrombosis or a reduction of an acute or non-acute embolism. In some embodiments, CFTR modulator improve one or more symptoms of thrombotic and/or thromboembolic events.

In one embodiment, CFTR modulator treatment improves thrombocyte hyperactivation to a non-pathological stage of thrombocyte function, particularly platelet activation.

In one embodiment, the effectiveness and physiological response of the treatment, as described herein, is determined by reduction of incidence of thrombotic and/or thromboembolic events and measuring thrombocyte activation as described herein.

In some embodiments, the medical condition is associated with a human pathogenic infection, such as a viral, bacterial, fungal or parasitic infection.

In one embodiment, pathological thrombocyte activation, thrombosis, coagulopathy, and/or a thromboembolic event occurs and/or persists in a human subject after a first onset of an infection with a human pathogen.

In one embodiment, the human subject has or had an infection with a human pathogen, such as a viral, bacterial, fungal or parasitic infection.

In one embodiment, the human subject has or had a viral infection selected from the group consisting of SARS-CoV-2 infection, SARS coronavirus infection, influenza virus infection, corona virus infection, respiratory syndrome infection, dengue virus infection, avian flu virus infection, swine flu virus infection, ebola virus infection, yellow fever virus infection, and entero virus infection, preferably a SARS-CoV-2 infection.

In one embodiment, the subject has or had a bacterial infection selected from the group consisting of legionella infection, chlamydia infection, streptococcus pneumoniae infection, haemophilus influenzae infection, staphylococcus aureus infection, escherichia coli infection, salmonella spp. infection, and neisseria meningitides infection.

In one embodiment, the human has or had a parasite infection selected from the group consisting of malaria infection including plasmodium falciparum infection, plasmodium malariae infection, plasmodium ovale infection, and plasmodium vivax infection.

In some embodiments, the subject had or is suspected to have had a fungal infection selected from the group consisting of aspergillus fumigatus infection, aspergillus flavus infection, candida infection (e.g., candida albicans), histoplasma capsulatum infection, and pneumocystis jirovecii infection.

In one embodiment, pathological thrombocyte activation, thrombosis, coagulopathy, and/or thromboembolic events occur independently of an infection.

In some embodiments, the onset of the infection is characterized by the presence of symptoms of said infection, and/or by detection of the infection in said subject comprising detection of components of the human pathogen, such as by a PCR-test, a flow cytometry analysis, an ELISA test, or an antigen-test.

In some embodiments, the infection is a respiratory viral infection, preferably a SARS coronavirus infection, more preferably a SARS-CoV-2 infection.

In some embodiments, the treatment comprises administering a CFTR modulator to a subject who has or had a respiratory viral infection or SARS coronavirus infection.

In some embodiments, the treatment comprises administering CFTR modulator to a subject who has or had a SARS-CoV-2 infection and said subject has a medical condition described herein, wherein said medical condition occurs or persists after the onset of first symptoms of SARS-CoV-2 infection.

It could not have been derived from the prior art that CFTR is a target for prevention or treatment of disease-associated hyperactivation of thrombocytes, such as pneumonia-associated hyperactivation of thrombocytes, and controlling hypercoagulative state.

The invention further relates to a cystic fibrosis transmembrane conductance regulator (CFTR) modulator for use in the treatment and/or prevention of a medical condition comprising atherosclerosis and pathological thrombocyte activation.

In one embodiment, the CFTR modulator is used to treat hypercoagulability/thrombophilia.

In some embodiments, the CFTR modulator is administered to a subject who has, is suspected to have or is at risk of having thrombocyte-mediated coagulopathy, such as hypercoagulability/thrombophilia.

In one embodiment, CFTR modulator treatment reduces atherosclerosis, pathological thrombocyte activation, such as thrombocyte hyperactivation, and/or hypercoagulability The CFTR modulator for use in an interventional therapy as described herein is intended to prevent, to encompass a slowing or reduction in disease progression comprising atherosclerosis, thrombocyte hyperactivation, and/or hypercoagulability or a reduction of an acute or non-acute atherosclerosis. In some embodiments, CFTR modulator improve one or more symptoms of atherosclerosis.

In one embodiment, the treatment using CFTR modulators reduces lethality for patients suffering from atherosclerosis. Of particular advantage is the reduction of the risk of death due to atherosclerosis by treatment with CFTR modulator, if not completely absent.

In one embodiment, the effectiveness and physiological response of the treatment, as described herein, is determined by measuring areas of severe narrowing, stenosis, as detectable by angiography, stress testing, Doppler ultrasound or computer tomography, and thrombocyte hyperactivation.

In some embodiments, the medical condition comprises a thrombocyte mediated stage of atherosclerosis disease.

For example, thrombocytes are involved at early and late stages of atherosclerosis involved in the development of atherosclerosis in the coronary or carotid arteries.

In one embodiment, the CFTR modulator is used to treat thrombocyte hyperactivation and/or hypercoagulability/thrombophilia in an early stage of atherosclerosis. In one embodiment, the CFTR modulator is used to treat thrombocyte hyperactivation and/or hypercoagulability/thrombophilia in a late stage of atherosclerosis. The early stage is preferably the first or initial stage in the development of atherosclerosis. The late stage is preferably the third stage in the development of atherosclerosis. Thrombocytes are typically involved in the pathogenesis of atherosclerosis at the first and the third stage.

In some embodiments, the CFTR modulator improve one or more symptoms of an early stage of atherosclerosis. In some embodiments, the CFTR modulator improve one or more symptoms of a late stage of atherosclerosis.

In one embodiment, the CFTR modulator is preferably administered for preventing development and/or progression of atherosclerosis at the first stage of atherosclerosis. In one embodiment, the CFTR modulator is preferably administered for the treatment of atherosclerosis at the third stage of atherosclerosis. CFTR modulators provide beneficial effects in both stages.

In some embodiments, the medical condition is selected from the group consisting of coronary artery disease, myocardial infarction, angina, stroke, transient ischemic attacks and peripheral artery disease.

In some embodiments, the medical condition comprises a thrombocyte-mediated vascular disease.

In some embodiments, the CFTR modulator is administered to a subject who has, is suspected to have or is at risk of having thrombocyte-mediated vascular disease. In one embodiment, thrombocyte hyperactivation and arterial thrombosis mediated vascular diseases comprise myocardial infarction, non-fatal stroke, peripheral vascular disease, and/or peripheral arterial disease, and vascular death.

In vascular disease, abnormal clotting occurs that can result in arterial disease, vein diseases, lymph diseases, blood flow disturbance, and ischemia. Thrombocyte dysfunction including hyperactivation mediates or increase the risk of a vascular disease, wherein said vascular disease can be selected from a group of aneurysm, atherosclerosis, peripheral artery disease, blood clots in veins, myocardial infarction, non-fatal stroke, peripheral vascular disease, and/or peripheral arterial disease, blood clotting disorders, and vascular death.

The CFTR modulator for use in an interventional therapy as described herein is intended to prevent, to encompass a slowing or reduction in disease progression comprising thrombocyte hyperactivation and arterial thrombosis mediated vascular diseases and vascular death. In some embodiments, CFTR modulator improve one or more symptoms of thrombocyte-mediated vascular disease, such as cyanosis, chest pain, heart attack, uncontrolled high blood pressure, heart failure, leg pain, and cramps.

In one embodiment, the effectiveness and physiological response of the treatment, as described herein, is measured by reduced incidence of non-fatal myocardial infarction, non-fatal stroke or vascular death in patients at increased risk of occlusive vascular events, and/or decrease in the need of cardiovascular surgery, such as bypass surgery or angioplasty.

In one embodiment, a CFTR modulator prevents from a thrombosis, embolism, and/or a pathological thrombocyte mediated atherosclerosis caused fatal outcome of a disease.

In some embodiments, the medical condition comprises pathological thrombocyte activation, adhesion and/or aggregation.

In some embodiments, the CFTR modulator is administered to a subject who has, is suspected to have or is at risk of having pathological thrombocyte activation, adhesion and/or aggregation.

Ca2+ influx mediates the shape change of activated platelets and their aggregation and adhesion. Ivacaftor significantly attenuates platelet aggregation in vitro. In Example 1, the inventors show that the use of a CFTR modulator reciprocally attenuated Ca2+ influx into thrombocytes via calcium channels. Ca2+ mediates both the shape change and secretion of activated thrombocytes, as well as their aggregation and adhesion. Surprisingly, a CFTR modulator can also significantly attenuate Ca2+-dependent aggregation (Figure 2). Pretreatment with a CFTR modulator reduced Ca2+-dependent agonist-induced aggregation of thrombocyte to a level comparable to that in healthy subjects. In a microfluidic in vitro disease model of damaged vessels, agonist-induced thrombus formation in the blood of COVID-19 patients was surprisingly significantly reduced in number and size to levels measured in the blood of healthy volunteers (Figure 2).

It could not have been derived from the prior art, that ivacaftor significantly attenuates platelet aggregation and calcium levels, even if a cell has been pre-treated with ivacaftor. The pretreatment experiments provide proof of concept for an advantageous and significant effect as preventive measures.

In one embodiment, treatment with a CFTR modulator reduces pathological thrombocyte activation, aggregation, and adhesion to physiological level compared to a healthy and non-affected control.

The CFTR modulator for use in an interventional therapy as described herein is intended to prevent, to encompass a slowing or reduction in disease progression comprising pathological thrombocyte aggregation, and adhesion or a prevention and/or reduction of an acute or non-acute thrombus formation In some embodiments, CFTR modulator improve one or more symptoms of thrombus formation, such as blockage of veins or arteries.

In one embodiment, the effectiveness and physiological response of the treatment, as described herein, is determined by measuring thrombocyte aggregation detected, for example, by aggregometry, and thrombocyte adhesion, detected, for example, by a microfluidic in vitro disease model of damaged vessels.

In some embodiments, the CFTR modulator is administered to a non-cystic fibrosis subject.

CFTR modulators have been developed for cystic fibrosis patients having a mutated form of the CFTR gene. CFTR modulators improve the expression and/or activity of the CFTR ion channel at the cell membrane.

It could not have been derived from the prior art that CFTR modulators also increase an activity of a non-mutated CFTR protein. The non-mutated CFTR is normally expressed, for example, in thrombocytes of non-cystic fibrosis patients comprising a physiological activity.

The skilled person could not have expected that CFTR modulators, such as the CFTR potentiator ivacaftor enhancing the activity of a mutant CFTR, would also improve the activity of a non-mutated CFTR protein. It is surprising that CFTR potentiator ivacaftor, can reduce or prevent activation in non-mutated CFTR protein, thus leading to the medical effect described herein.

In some embodiments, the CFTR modulator is administered to a subject suffering additionally from cystic fibrosis.

Cystic fibrosis is a severe genetic disorder primarily affecting lungs, but also the pancreas, liver, kidneys and intestines. Long-term problems include lung infections, difficulty in breathing, coughing up mucus. Cystic fibrosis often leads to early death.

In one embodiment, a CFTR modulator is administered to a cystic fibrosis patient who has, is suspected to have or is at risk of developing thrombosis, thrombocytopathies, coagulopathies, and/or thromboembolic events. As shown in Example 2, cystic fibrosis patients treated with CFTR modulator provide beneficial effects on risks of developing thrombosis, thrombocytopathies, coagulopathies, and/or thromboembolic events compared to untreated cystic fibrosis patients.

Remarkably, a retrospective analysis of multicentric patient data (TriNetX network) of CF patients affected from COVID-19, showed better outcome in CF patients upon SARS-CoV-2 infection when on CFTR-modulator therapy.

Treatment using CFTR modulators is particularly beneficial in reducing pathological thrombocyte activation and risks of developing thrombosis, thrombocytopathy, coagulopathiy, and/or thromboembolic events in cystic fibrosis and non-cystic fibrosis patients.

In some embodiments, the CFTR modulator is a CFTR activator, CFTR potentiator, CFTR corrector or CTFR amplifier, preferably a CFTR potentiator or a CFTR corrector.

In some embodiments, the CFTR modulator is selected from the group consisting of ivacaftor, lumacaftor, tezacaftor and elexacaftor.

In one embodiment, one or more CFTR modulators can be administered to human subject.

In one embodiment, the CFTR modulator is administered for the treatment of thromboses and embolisms to human subjects suffering from community-acquired pneumonia, hospital acquired pneumonia und COVID-19.

In one embodiment, the CFTR modulator is administered for the prevention of thromboses and embolisms to human subjects suffering from community-acquired pneumonia, hospital acquired pneumonia und COVID-19.

In one embodiment, the CFTR modulator is administered for the treatment of thromboses and embolisms to human subjects suffering from inflammatory and/or infectious diseases, such as sepsis and pneumonia.

In one embodiment, the CFTR modulator is administered for the treatment and/or prevention of thromboses and embolisms to human subjects suffering from thrombocyte-mediated vascular disease, such as atherosclerosis, peripheral vascular disease, myocardial infarction, stroke, and vascular death.

As shown in Example 2, treatment of cystic fibrosis patients with a CFTR modulator reduced the risk of coronary heart disease of 63.7% and an atherosclerosis of 73.9%. It could not have been derived from the prior art that CFTR modulators, including ivacaftor, lumacaftor, tezacaftor or elexacaftor, have significantly beneficial effects on coronary heart disease and atherosclerosis.

In one embodiment, the CFTR modulator is ivacaftor.

In one embodiment, ivacaftor is administered for the treatment and/or prevention of thromboses and embolisms to human subjects suffering from community-acquired pneumonia, hospital acquired pneumonia und COVID-19.

In some embodiments, comprising the treatment of a medical condition comprising thrombosis in a subject suffering from a SARS coronavirus infection, preferably a SARS-CoV-2 infection, wherein the CFTR modulator is ivacaftor.

In one embodiment, the treatment comprises administering ivacaftor to a subject who has or had a SARS-CoV-2 infection and said subject has a medical condition comprising thrombosis, thrombotic event, pneumonia-associated hyperactivation of thrombocytes, thrombocytopathy, and/or coagulopathy.

In some embodiments, symptoms of infection with a SARS-virus are fever, sore throat, cough, myalgia or fatigue, and in some embodiments, additionally, sputum production, headache, hemoptysis and/or diarrhea. In some embodiments, symptoms of an infection with a SARScoronavirus, for example SARS-CoV-2, are fever, sore throat, cough, lack of taste and/or smell, shortness of breath and/or fatigue.

In one embodiment, the CFTR modulator reduces the degree of thrombosis with microangiopathy and prevents subjects from fatal thromboembolic events.

In some embodiments, a dosage and frequency of said CFTR modulator, preferably ivacaftor, comprises:
(a) A dosage of 2-2000 mg/day, preferably at 20-1000 mg/day, more preferably at 100-200 mg/day to a human subject up to 30kg body weight, and 200-400 mg/day to a human subject above 30kg body weight, and/or
(b) A frequency of 2 to 4 times per day, preferably 2 times daily, wherein the route of administration is subcutaneously, intravenously or orally, preferably orally, via a tablet of 1 mg to 1000 mg per dose, more preferably 10 mg to 500 mg per dose, even more preferably 50 mg to 100 mg per dose to a human subject up to 30 kg body weight, and 100 mg to 200 mg per dose to a human subject above 30 kg body weight.

In one embodiment, a therapeutically effective amount of at least one CFTR modulator is administered to a human subject in need thereof.

In some embodiments, ivacaftor is used in the treatment of chronic thrombosis and/or atherosclerosis in a human subject, wherein ivacaftor is administered at a daily dose of 100-200 mg/day to a human subject up to 30kg body weight, and 200-400 mg/day to a human subject above 30kg body weight.

In some embodiments, one or more CFTR modulators are administered to a human subject for use in the treatment of chronic thrombosis and/or atherosclerosis in said subject.

In some embodiments, the treatment comprises administration of a CFTR modulator 2-4 times daily for at least one week, at least one month, at least year or livelong, preferably 2-4 times daily, for example, for 1 month to a non-cystic fibrosis subject or livelong to a cystic fibrosis subject, wherein the route of administration is preferably oral, preferably 50 mg to 100 mg per dose via oral delivery up to 30 kg body weight, and 100 mg to 200 mg per dose via oral delivery above 30 kg body weight.

In preferred embodiments, the compounds are administered at concentrations or amounts, or according to dosage regimes, already established in the art, such as those for which regulatory approval has been issued (e.g. by the FDA or EMA), or in doses currently being assessed during phase 2 clinical trials, and/or according to the maximum allowed dose according to a phase I trial.

Some embodiments, regarding the particular relative amounts mentioned herein, are based on clinically allowed or currently trialed doses of the various compounds described herein, being combined into a pharmaceutical composition as described herein.

In some embodiments, CFTR modulators improve one or more symptoms of thrombosis and/or atherosclerosis, as described herein. For example, a thrombocyte hyperactivation, hypercoagulability/thrombophilia, pathological adhesion and/or aggregation may be effectively treated by the administration orally at a daily dose about 2-2000 mg/day, preferably at 20-1000 mg/day, more preferably at 100-200 mg/day to a human subject up to 30kg body weight, and 200-400 mg/day to a human subject above 30kg body weight, per day.

In one embodiment, the treatment, dosage and/or frequency of CFTR modulator administration is configured to reduce calcium influx in target cells, preferably in thrombocytes.

In one embodiment, the treatment, dosage and/or frequency of CFTR modulator administration is configured to reduce pathological thrombocyte activation, adhesion and/or aggregation.

In one embodiment, the treatment, dosage and/or frequency of CFTR modulator administration is configured to reduce a level of thrombocyte activation to a level, or below a level, present prior to treatment.

In one embodiment, the level is determined in a sample comprising and/or derived from a bodily fluid and/or cells from the subject, preferably from blood, serum or plasma. Preferably, samples are collected and analysed as described in Example 1.

In one embodiment, said treatment is administered at a dosage and frequency configured to prevent pathological thrombocyte activation, adhesion and/or aggregation.

In one embodiment, said treatment is administered at a dosage and frequency configured to reduce a level of pathological thrombocyte activation, a level of adhesion, a level of aggregation, and/or to reduce a level of thrombocyte activation to a level of at least 5%, 10%, 20%, 30%, 40% or at least 50% present prior to treatment.

In one embodiment, said treatment is administered at a dosage and frequency configured to prevent and/or to reduce a level of calcium influx and/or a surface expression of thrombocyte activation marker to a level of at least 20% below a level at treatment initiation, wherein said of thrombocyte activation markers include CD62p, CD63, and/or CD41/CD61.

In one embodiment, calcium influx, surface expression of a thrombocyte activation marker, thrombocyte activation, thrombocyte adhesion, thrombocyte aggregation, and intracellular calcium levels are determined as described in the Examples. Other means are known to one skilled in the art.

A skilled person is capable, based on the experimental and written support provided herein, to carry out the invention, in combination with common knowledge in the art. By employing the quantitative measures described in the examples, or by using alternative quantitative means for determining reductions in levels of one or more thrombocyte activation, adhesion and/or aggregation, the combination of pharmaceutical compounds and their necessary doses can be determined and adjusted to obtain the beneficial effects as described herein.

In one embodiment, the CFTR modulator is solely administered to the subject described herein. In one embodiment, the CFTR modulator is administered in combination with a non-CFTR modulator to the subject described herein.

The invention further relates to a pharmaceutical composition comprising a CFTR modulator for use in the treatment and/or prevention of a medical condition as described herein, wherein said CFTR modulator is in admixture with a pharmaceutically acceptable carrier and/or formulated in a pharmaceutically buffered solution.

In another aspect, the invention relates to a pharmaceutical composition comprising a CFTR modulator for use in the treatment of thrombosis and/or atherosclerosis associated with pathological thrombocyte activation in a human subject.

The composition provided by the present disclosure may be capable of delaying or stopping the progression and/or persistence of a medical condition comprising thrombosis and/or atherosclerosis, as described herein. For example, a thrombocyte hyperactivation, hypercoagulability/thrombophilia, pathological adhesion and/or aggregation. The use of samples received from COVID-19 patients and controls in three cohorts is an advantage of the present disclosure and a strength of the study. The present disclosure is the first to show an association with CFTR activity and thrombosis and/or atherosclerosis, comprising thrombocyte hyperactivation, hypercoagulability/thrombophilia, pathological adhesion, and/or aggregation in a human subject, and providing means to treat said disease.

Further examples of pharmaceutical compositions, medical conditions, treatments, subjects, and targets are described in detail below.

The features of the invention regarding methods of treatment and diagnosis, and descriptions of the composition comprising CFTR modulator for use in the treatment of various medical conditions, apply to the composition itself, and vice versa. Any of the features disclosed herein for any given embodiment are applicable to other embodiments, as deemed reasonable by a skilled person.

### DETAILED DESCRIPTION OF THE INVENTION

Singular expressions should be understood to include the concept in the plural form, unless explicitly stated otherwise. Thus, singular articles (e.g., "a," "an," "the," and the like in English) should also be understood to include the concept in the plural form, unless specifically noted otherwise. Further, terms used herein shall be understood to have the meaning generally accepted in the art, unless otherwise expressly noted.

### Cystic fibrosis transmembrane conductance regulator (CFTR)

The "Cystic fibrosis transmembrane conductance regulator" or "CFTR" is a protein forming an anion channel that is found at the apical membrane of numerous secretory cells. It consists of 1480 amino acids and is assigned to the ATP-binding cassette (ABC) transporters. The channel consists of 5 domains: 2 transmembrane omens (TMD1 and TMD2), each with 6 hydrophobic segments (M1-6 and M7-12), form the channel pore, a regulatory domain (R) initiates opening, and 2 nucleotide-binding domains (NBD1 and NBD2) finally induce channel opening. The opening is cAMP (cyclic adenosine monophosphate) dependent: The regulatory domain is phosphorylated by a protein kinase after cAMP activation, followed by ATP binding to the two NBDs. NBD1 and NBD2 subsequently dimerize, causing the channel pore to open. Hydrolysis of the ATP closes the channel again. In addition to its function as an ion channel, the CFTR protein is also involved in the regulation of other channels, such as the ENaC (epithelial sodium channel), ROMK1 and ROMK2 (potassium channels), or ORCCs (outwardly rectifying chloride channels), and interacts with other proteins. Expression of the CFTR gene is tissue-specific, and the protein is found in high proportions in the intestinal mucosa and the biliary system, but also in the pancreas, lungs, sweat glands, and reproductive organs.

To date, more than 2100 mutations of the CFTR gene have been described and 382 of these are currently classified as causing cystic fibrosis (https://cftr2.org/mutations_history).

"Cystic fibrosis" is an autosomal recessive life-shortening multi-organ disease. CF results from a mutational defect in the CFTR (cystic fibrosis transmembrane conductance regulator) protein, an anion channel of exocrine glands. In Europe, about 45,000 patients are currently included in a registry. This results in a prevalence in Europe of 8.4/100,000 population (p.e.) on average, with a significant geographic variation from 1.0/100,000 p.e. in Latvia to 29.8/100,000 p.e. in Ireland. In Germany, the incidence is currently estimated at 1:3300 to 1:4800 newborns. In the United States, approximately 30,000 patients are currently registered. CFTR mutations are functionally classified into 6 mutation classes:
- Class I: Includes mutations that do not result in the production of CFTR protein.
- Class II: Includes mutations that cause defective protein folding and consequent retention in the endoplasmic reticulum and consecutive premature proteasomal degradation.
- Class III: Include mutations in which a CFTR channel is expressed at the cell membrane and exhibits a regulatory disorder with significantly reduced opening probability.
- Class IV: Includes mutations that result in significantly reduced ion conductance of the CFTR channel expressed at the apical cell membrane.
- Class V: Includes mutations that decrease the quantity of functional CFTR channels.
- Class VI: Includes mutations that result in markedly impaired membrane stability of the functional CFTR protein, resulting in premature protein degradation.

Decreased function of the CFTR channel leads to a reduction in apical chloride secretion or reabsorption in the sweat gland. In some tissues, such as the respiratory tract, there is also hyperactivity of apical ENaC with consequent hyper-resorption of sodium ions from the lumen. As a result, interstitial water influx into the lumen of the glands is limited, resulting in hyperviscous secretions and also leads to inflammation. These in turn obstruct the lumen and the excretory ducts of exocrine glands. In the airways, decreased hydration of the so-called airway surface liquid (ASL) leads to impaired mucocilliary clearance. Bicarbonate is also secreted via the CFTR channel. Decreased CFTR function correspondingly leads to a decrease in the pH of secretions. Animal models show that acidification in the airways leads to inhibition of endogenous antimicrobial peptides. Organ systems affected include, in particular, the respiratory tract, the digestive tract, and the reproductive organs. CF is the most common genetic disease in the Caucasian population. Symptoms of CF comprise nasal polyps, chronic sinusitis, anosmia, recurrent bronchopulmonary infections, bronchial obstruction, atelectasis, infections caused by gram-negative bacteria such as Pseudomonas aeruginosa, by Staph. aureus and Haemophilus influenzae or by fungi, bronchiectasis, allergic bronchopulmonary aspergillosis, pulmonary hyperinflation, hemoptysis, pneumothorax, respiratory failure, dyspnea, cor pulmonary hypertension, exocrine pancreatic insufficiency, recurrent or chronic pancreatitis, type 3 diabetes mellitus, meconium ileus, failure to thrive, rectal prolapse, distal intestinal obstruction syndrome, biliary cirrhosis, fibrosis, primary sclerosing cholangitis, cholelithiasis, osteoporosis, arthropathy, salt wasting syndrome.

### CFTR modulator

As used herein, the term "CFTR-Modulator" refers to its common meaning in the art, and is understood as an agent or compound for modulating, preferably improving or restoring, the function of the CFTR protein, preferably the channel function of CFTR protein. The CFTR protein is known as an ion channel for chloride ions allowing chloride ions to pass across the cell membrane; for example, to increase intracellular chloride ion concentrations. In cells, organs or organisms with defective CFTR protein caused by one ore more mutations within CFTR gene or any nucleic acid region controlling/modulating CFTR gene expression, the chloride ion channel can be absent, disturbed or non-functional.

CFTR protein functioning is determined by measuring chloride flow and/or intracellular chloride level can by using methods known in the art, for example by measuring single channel activity by patch clamp technique, short circuit currents in Ussing chambers, or changes in intracellular Cl levels by CI- sensitive fluorescent dyes as a function of extracellular Cl-concentrations.

As described herein, a defect of CFTR protein function results in a decreased chloride flux across the cell membrane. CFTR modulators have been shown to effectively target specific disturbances in the CFTR protein and/or CFTR protein levels. CFTR modulators are preferably used to improve the function of the CFTR protein being disturbed and/or CFTR protein levels. As described herein, CFTR modulator is preferably used to treat and/or prevent medical conditions, described herein, associated with CFTR protein function, wherein CFTR protein is a non-mutated CFTR protein. The CFTR modulator used to treat and/or prevent medical conditions described herein associated with CFTR protein function, wherein CFTR protein is a mutated CFTR protein associated with cystic fibrosis.

CFTR modulators have been developed for use in the treatments of cystic fibrosis, in particular for different functional disturbances associated with one or more mutation classes as described herein. The first CFTR modulators were identified by high-throughput functional screening (HTS) of a large number of chemical compounds on cell cultures with different CFTR mutations. Various compounds acting as CFTR modulator are known in the prior art. (Table 1). The CFTR modulator can be a CFTR activator, a CFTR potentiator, a CFTR corrector or a CTFR amplifier, preferably a CFTR potentiator or a CFTR corrector.

The term "potentiator" refers to an agent or to a compound that enhances the action of a target structure, such as a protein. The CFTR protein is shaped like a tunnel that can be closed by a gate. CFTR potentiators are expected to enhance the functionality of CFTRs already expressed at the apical cell membrane. For example, CFTR potentiators increase the channel opening or gating probability of the disturbed CFTR protein while being located at the membrane and have gating (Class III) and/or conductance (Class IV) mutations. That can be determined again by determining the chloride flux.

In embodiments, the CFTR potentiator can be selected from, without limitation, Ivacaftor (CAS No.: 873054-44-5, commercially available under the tradename Kalydeco® by Vertex Pharmaceuticals), GLPG2451 (CAS No.: 2055015-61- commercially available from Galapagos NV) and GLPG1837 (CAS No.: 1654725-02-6 commercially available from Galapagos NV), QBW251 10 (commercially available from Novartis), PTI-808 (commercially available from Proteostasis Therapeutics), FDL176 (commercially available from Flatley Discovery Lab), 4,6,4'-trimethylangelicin (TMA, a synthetic derivative of angelicin), CFpot-532, CoPo-22 or derivatives or pharmaceutically acceptable salts thereof.

The term "corrector" refers to an agent or to a compound that improves or fixes assembly of a target structure, for example correct folding of a protein.

The CFTR correctors are expected to improve correct folding of the nascent amino acid chain translated from a mutated CFTR gene into its correct three-dimensional structure, trafficking of the protein product expressed by a mutated CFTR gene to the cell membrane, and/or stability of the protein product of the mutated CFTR gene at the cell membrane.

CFTR correctors are expected to enable and/or expand the incorporation of CFTR protein into the cell membrane. This leads to more CFTR protein at the cell membrane, even with impaired protein function. CFTR protein function can be determined by measuring the chloride flux across the cell membrane.

CFTR correctors have been developed for cystic fibrosis patients having the F508del mutation on at least one CFTR gene allele, and wherein such mutation prevents the CFTR protein from forming a functional three dimensional shape (Class II mutation).

In embodiments, the CFTR corrector can be selected from, without limitation, Lumacaftor (CAS No.: 936727-05-8, VX-809, commercially available from Vertex Pharmaceuticals), C18 (VRT-534) (an analogue of Lumacaftor), VRT-768, VRT-325, Tezacaftor (CAS No.: 1152311-62-0, VX-661, commercially available from Vertex Pharmaceuticals), Elexacaftor (CAS No.: 2216712-66-0, VX-445, commercially available from Vertex Pharmaceuticals), Bamocaftor (CAS No.: 2204245-48-5, VX 659 commercially available from Vertex Pharmaceuticals), Bamocaftor potassium (CAS 2204245-47-4, VX 659 potassium salt commercially available from Vertex Pharmaceuticals) Posenacaftor (CAS No.: 2095064- 05-2, PTI-801, commercially available from Proteostasis Therapeutics), Cavosonstat (CAS No.: 1371587-51-7, N91115, commercially available from Nivalis Therapeutics), GLPG2222 (CAS No.: 1918143-53-9, commercially available from Galapagos NV), GLPG2737 (commercially available from Galapagos NV), or derivatives, or pharmaceutically acceptable salts thereof.

The term "amplifier" refers to an agent or to a compound augmenting activity of a corrector or potentiator by enhancing the number of target structures being available for said corrector or potentiator, for example an increase in protein levels by improving mRNA translation.

CFTR amplifiers also augment the activity of other CFTR modulators by stabilizing CFTR mRNA during translation, allowing more CFTR protein to be expressed and available for correctors and potentiators to act upon.

The CFTR amplifier is expected to increase the amount of mutant CFTR messenger RNA, and consequently the amount of CFTR protein. The amount of messenger RNA can be determined by methods known in the art, qRT-PCR, micro-array, RT-PCR, or shot-gun sequencing. CFTR amplifiers are designed for CFTR mutations resulting in insufficient CFTR protein expression. CFTR amplifiers are a class of drugs that increase CFTR protein levels in cells and tissues. As CFTR amplifiers increase the amount of mutant CFTR messenger RNA, and consequently the amount of CFTR protein, they thereby increase the substrates for other CFTR modulators. Amplifiers by themselves do not correct or improve the function of the CFTR protein.

The CFTR amplifier can be selected from, without limitation, Nesolicaftor (PTI-428, commercially available from Proteostasis Therapeutics) and PTI- 5 CH (Kenneth A. Giuliano et. al., SLAS Discov. 2018 Feb; 23(2): 111-121) or derivatives, or pharmaceutically acceptable salts thereof.

CFTR modulators are also administered in combinations, such as a CFTR corrector combined with one or more CFTR amplifier and/or one or more CFTR potentiators. For example, a combination of lumacaftor and ivacaftor (approved as Orkambi by FDA and EMA) or of tezacaftor and ivacaftor (approved as Symdeko by FDA), or a combination of elexacaftor, tezacaftor and ivacaftor (approved as Kaftrio by FDA) can be used for treatment.

**Table 1 - Structural Formulas of CFTR modulators**

| | | |
|---|---|---|
| 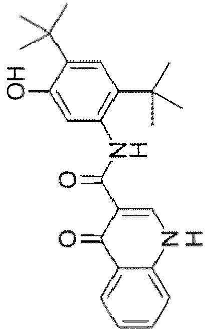 | 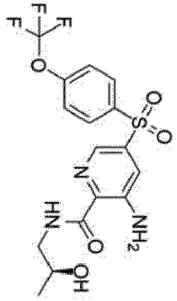 | 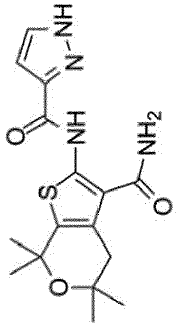 |
| Ivacaftor (CAS No.: 873054-44-5, Vertex harmaceuticals) is N-(5-hydroxy-15 2,4-ditert-butylphenyl)-4-oxo-1 H-quinoline-3-carboxamide | GLPG2451 (CAS No.: 2055015-61-5, Galapagos NV,) | GLPG1837 (CAS No.: 1654725-02-6, Galapagos NV) is N-(3-carbamoyl-5,5,7,7-tetramethyl-4,7-dihydro-5H-thieno[2,3-c]pyran-2-yl)-1H-pyrazole-3-carboxamide |
| 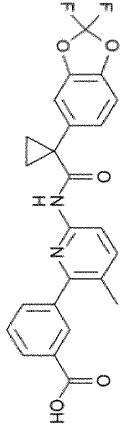 | 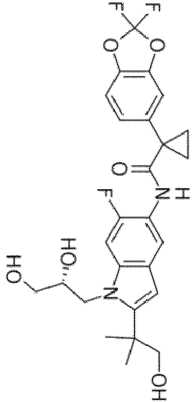 | 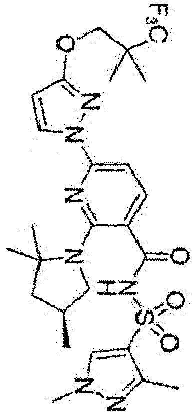 |
| Lumacaftor (CAS No.: 936727-05-8, VX 809, Vertex Pharmaceuticals) is 3-20 [6-({[1-(2,2-difluoro-1,3-benzodioxol-5-yl)cyclopropyl]carbonyl}amino)-3-methylpyridin-2-yl]benzoic acid | Tezacaftor (CAS No.: 1152311-62-0 Vertex Pharmaceuticals) is 1-(2,2-difluoro-1,3-benzodioxol-5-yl)-N-[1-[(2R)-2,3-dihydroxypropyl]-6-fluoro-2-(1-hydroxy-2-methylpropan-2-yl)indol-5-yl]cyclopropane-1-carboxamide | Elexacaftor (CAS No.: 2216712-66-0 Vertex Pharmaceuticals) is N-(1,3-dimethylpyrazol-4-yl)sulfonyl-6-[3-(3,3,3-trifluoro-2,2-dimethylpropoxy)pyrazol-1-yl]-2-[(4S)-2,2,4-trimethylpyrrolidin-1-yl]pyridine-3-carboxamide |
| 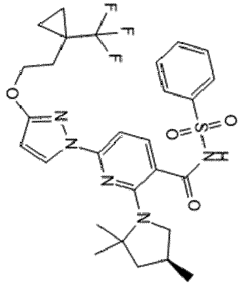 | 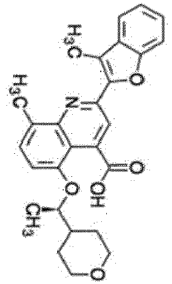 | 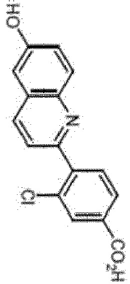 |
| Bamocaftor (CAS No.: 2204245-48-5, Vertex Pharmaceuticals) is (S)-N-(phenylsulfonyl)-6-(3-(2-(1-(trifluoromethyl)cyclopropyl)etho xy)-1Hpyrazol-1-yl)-2-(2,2,4-trimethylpyrrolidin-1-yl)nicotinamide; Bamocaftor potassium (CAS 2204245-47-4, Vertex pharamceuticals) is Potassium (benzenesulfonyl)[6-(3-[2-[1-(trifluoromethyl)cyclopropyl]etho xy]-1 H-pyrazol-1-yl)-2-[(4S)-2,2,4-trimethylpyrrolidin-1-yl]pyridine-3-carbonyl]azanide | Posenacaftor (CAS No.: 2095064-05-2, PTI-801 Proteostasis Therapeutics) 15 is 8-methyl-2-(3-methyl-1-benzofuran-2-yl)-5-[(1R)-1-(oxan-4-yl)ethoxy]quinoline-4-carboxylic acid | Cavosonstat (CAS No.: 1371587-51-7, N91115, Nivalis Therapeutics) is 3-Chloro-4-(6-hydroxy-2-quinolinyl)benzoic acid |
| 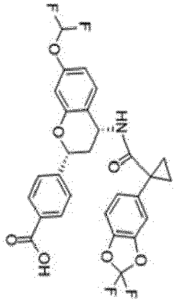 | 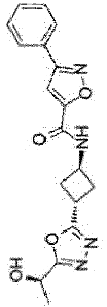 | 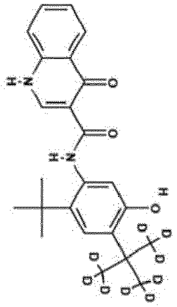 |
| GLPG2222 (CAS No.: 1918143-53-9, Galapagos NV) is 4-[(2R,4R)-4-[[1-(2,2-difluoro-1,3-benzodioxol-5-yl)cyclopropanecarbonyl]amino]-7-(difluoromethoxy)-3,4-dihydro-2H-chromen-2-yl]benzoic acid | Nesolicaftor (PTI-428, Proteostasis Therapeutics); CAS 1953130-87-4 | Deutivacaftor (CAS No.: 1413431-07-8, deuterated Ivacaftor or VX 561 commercially available from Vertex Pharmaceuticals) is N-[2-tert-butyl-4-[1,1,1,3,3,3-hexadeuterio-2-(trideuteriomethyl)propan-2-yl]-5-hydroxyphenyl]-4-oxo-1H-quinoline-3-carboxamide |
| 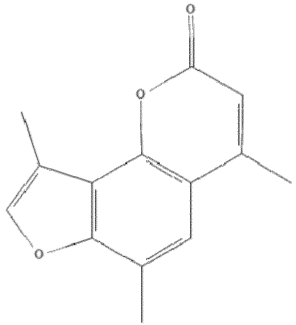 | 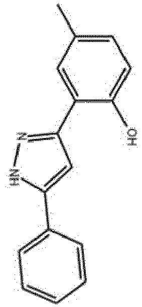 | 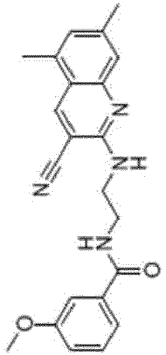 |
| 4,6,4'-trimethylangelicin; CAS: 90370-29-9 4,6,9-trimethylfuro[2,3-h]chromen-2-one | CFpot-532; VRT-532;CFpot 532;VRT-532; 4-METHYL-2-(5-PHENYL-1H-PYRAZOL-3-YL)PHENOL;PHEN OL, 4-METHYL-2-(5-PHENYL-1H-PYRAZOL-3-YL)- | CoPo-22, CAS [606101-83-1] |
| 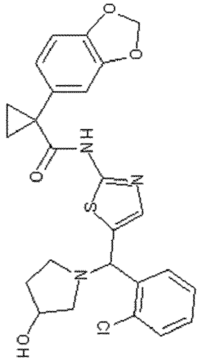 | 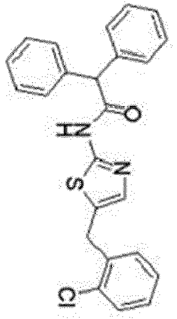 | 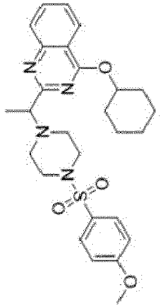 |
| C18 (VRT-534), CF-106951, CAS: 862574-51-4, 1-(benzo[d][1,3]dioxol-5-yl)-N-(5-((2-chlorophenyl)(3-hydroxypyrrolidin-1-yl)methyl)thiazol-2-yl)cyclopropanecarboxamide | VRT-768 | VRT-325, VRT-325, VRT 325, VRT325; CFcor 325; CFcor-325; CFcor325; CAS#: 815592-21-3; 4-(cyclohexyloxy)-2-(1-{4-[(4-methoxybenzene)sulfonyl]pipera zin-1-yl}ethyl)quinazoline |

### Hemostasis, thrombocyte activation, adhesion and/or aggregation

Thrombocytes are blood cells that play important roles in blood clotting and the closure of blood vessel injuries. In circulating blood, thrombocytes are in an inactive state. In the event of injury, and subsequent opening of blood vessels, hemostasis occurs within a short time due to adhesion and aggregation of platelets and subsequent fibrin formation. Surface contact results in thrombocyte activation. Thrombocytes activation includes adhesion, shape change, aggregation, and release reaction. Activators such as ADP, collagen, thromboxane and thrombin cause thrombocytes to change shape and thrombocytes aggregation occurs.

Although thrombocyte activation is an important, natural element of blood clotting and vascular occlusion in the body, platelet activation is also associated with a variety of diseases, including thrombosis, vascular disease, and embolism.

"Hemostasis" also refers to a biological process that stops the bleeding that occurs when blood vessels are injured. Further leakage of blood from the bloodstream is prevented, which is a prerequisite for wound healing. Hemostasis must be confined to the area of injury and not triggered erroneously by other events such as inflammation or infection. Hemostasis can be divided into two sub-processes, which, however, interact with each other.

Under non-pathological physiological conditions, in primary hemostasis, thrombocytes are activated by the process of thrombocyte adhesion. The release of calcium-lonen, ADP, Serotonin, Thromboxan A2 also induces coagulation. Thrombocytes aggregate for the formation of a white thrombus that temporarily closes a wound. These mechanisms promote activation and aggregation of additional thrombocytes. Secondary hemostasis, i.e., phase of blood coagulation, includes an activation phase comprising activation of thrombocytes and formation of active thrombin, and a coagulation phase. A white thrombus of primary hemostasis is transformed into a red thrombus. Therefore, thrombocyte dysfunction may be characterized by pathological aggregation, proliferation, or adhesion of thrombocytes.

In one embodiment, the pathological thrombocyte activation is also a thrombocyte dysfunction. In one embodiment, the pathological thrombocyte activation comprises thrombocyte hyperactivation, pathological thrombocyte aggregation, pathological thrombocyte adhesion, and/or pathological thrombocyte-mediated coagulation, such as hypercoagulability. "Hypercoagulability" is the increased clottability of blood.

### Medical indications and conditions

In one aspect of the present invention there is provided a pharmaceutical composition comprising one or more CFTR modulators for use in the treatment of a medical condition comprising thrombosis, thrombotic- and/or thromboembolic event.

In one aspect of the present invention there is provided a pharmaceutical composition comprising one or more CFTR modulators for use in the treatment of a medical condition comprising atherosclerosis and pathological thrombocyte activation.

A "medical condition" refers to any impairment of a normal physiological function of a cell, tissue part, organ, system of an organism, or organism resulting from various causes, such as infection, inflammation, environmental factors, or genetic defect, and characterized by an identifiable group of signs, symptoms, or both.

A medical condition treated or prevented using a CFTR modulator include, without limitation, thrombosis, thrombotic event, embolism, thromboembolism, hypercoagulative state, atherosclerosis, intravascular coagulation, thrombotic microangiopathy, pneumonia-associated hyperactivation of thrombocytes, pathological thrombocyte hyperactivation, pathological thrombocyte aggregation, and/or pathological thrombocyte adhesion.

A "disease" or "disorder" refer to any condition of the body or any of its parts being impaired or disturbed of its normal physiological functioning and is typically manifested by distinguishing signs and symptoms.

Disease and/or disorders treated or prevented using a CFTR modulator include, without limitation, pulmonary disorders, thrombocytopathy, coagulopathy, coronary artery disease, arteriosclerosis, myocardial infarction, angina, stroke, transient ischemic attacks, peripheral artery disease, infectious disease, CAP, HAP, COVID-19, systemic or extrapulmonary infectious, inflammatory diseases, rheumatic diseases, such as systemic lupus erythematosus or Schönlein-Henoch purpura, hematologic diseases, such as thrombotic thrombocytopenic purpura, cardiovascular diseases, such as artheriosclerosis, stroke, myocardial infarction, or acute or chronic pulmonary thromboembolism. The medical condition described herein, is preferably associated with pathological thrombocyte activation, adhesion and/or aggregation.

In one aspect of the present invention there is provided a pharmaceutical composition comprising one or more CFTR modulators for use in the treatment of a medical condition comprising pulmonary disorders.

Pulmonary disorders, can be selected from, without limitation, pulmonary hypertension, secondary pulmonary disorders, secondary pulmonary hypertension, associated with or caused by, respectively, certain primary disorders such as artherosclerosis, cardiovascular diseases (CVD such as coronary artery diseases (CAD) (angina and myocardia infarction (commonly known as a heart attack)), stroke, heart failure, hypertensive heart disease, rheumatic heart disease, cardiomyopathy, abnormal heart rhythms, congenital heart disease, valvular heart disease, carditis, aortic aneurysms, peripheral artery disease, thromboembolic disease, and venous thrombosis.

Human subjects with pathological thrombocyte activation, aggregation, and/or adhesion, atherosclerosis, thrombosis, thromboembolism, have specific groups of signs and symptoms that are classified as a specific disease or disorders described herein.

As used herein, a "patient with symptoms" a disease or disorder, described herein, is a subject who presents with one or more of that disease or disorder. As used herein, a "patient that is at risk of developing" a disease or disorder, described herein, relates to a subject, preferably distinct from any given person in the general population, who has an increased (e.g. above-average) risk of developing disease or disorder.

As used herein, a "patient with symptoms of an infectious disease" is a subject who presents with one or more of, without limitation, fever, diarrhea, fatigue, muscle aches, coughing, if have been bitten by an animal, having trouble breathing, severe headache with fever, rash or swelling, unexplained or prolonged fever or vision problems. Other symptoms may be fever and chills, very low body temperature, decreased output of urine (oliguria), rapid pulse, rapid breathing, nausea and vomiting. In preferred embodiments the symptoms of an infectious disease are fever, diarrhea, fatigue, muscle aches, rapid pulse, rapid breathing, nausea and vomiting and/or coughing. As used herein "infectious disease" comprises all diseases or disorders that are associated with bacterial and/or viral and/or fungal and/or parasite infections.

As used herein, a patient with "symptoms of a viral infection of the respiratory tract" is a subject who presents with one or more of, without limitation, cold-like symptoms or flu-like illnesses, such as fever, cough, runny nose, sneezing, sore throat, having trouble breathing, headache, muscle aches, fatigue, rapid pulse, rapid breathing, nausea and vomiting, lack of taste and/or smell and/or malaise (feeling unwell).

As used herein, the term "a patient that is at risk of developing a severe acute respiratory syndrome (SARS)" relates to a subject, preferably distinct from any given person in the general population, who has an increased (e.g. above-average) risk of developing SARS. In some embodiments, the patient has symptoms of SARS or symptoms of a SARS Coronavirus infection. In some embodiments, the patient has no symptoms of SARS or symptoms of a SARS Coronavirus infection. In some embodiments, the subject has been in contact with people with SARS Coronavirus infections or symptoms. In some embodiments, the person at risk of developing SARS has been tested for the presence of a SARS Coronavirus infection. In some embodiments, the person at risk of developing SARS has tested positive for the presence of a SARS Coronavirus infection, preferably a coronavirus infection.

In some embodiments, the patient that has or is at risk of developing a severe acute respiratory syndrome (SARS) has a coronavirus infection.

### Thrombosis, thrombotic- and/or thromboembolic event

A "thrombosis" or "thrombotic event" is the formation of a blood clot in a blood vessel that obstructs the flow of blood through the circulatory system. Even if a blood vessel is not injured, blood clots can form in the body under certain conditions.

A "thromboembolism" refers to a blockage of another blood vessel by a blood clot (thrombus), whereby the blood clot has formed in one blood vessel, breaks loose, is carried away by the blood stream, and blocks the other blood vessel.

An "embolus" is a clot or part of a clot that breaks loose and spreads throughout the body.

"Coagulopathy" or" blood clotting disorder" refers to a disorder of blood clotting (hemostasis). An increased tendency to bleed (hemorrhagic diathesis) with decreased blood clotting is called minus coagulopathy, and increased blood clotting (hypercoagulability) is called plus coagulopathy. Coagulopathies, by definition, are caused by a deficiency or dysfunction of clotting factors.

A "thrombocyte dysfunction" refers any problem in a thrombocyte itself or to an external factor altering the function of normal thrombocytes.

A "thrombocytopathy" refers to any of several blood disorders characterized by platelet (thrombocyte) dysfunction resulting in prolonged bleeding time, defective clot formation, and a tendency to bleed. Hereditary plateletopathies include von Willebrand disease, thrombasthenia, which is characterized by abnormal retraction of blood clots and impaired platelet aggregation, and Bernard-Soulier syndrome, which is characterized by abnormally large platelets. In addition, plateletopathy is sometimes seen in Down syndrome and Wiskott-Aldrich syndrome (an immune disorder).

Thrombosis can occur in veins (venous thrombosis) or in arteries (arterial thrombosis). Venous thrombosis results in congestion of the affected part of the body, while arterial thrombosis (and less sever venous thrombosis) affects the blood supply and results in damage to the tissue supplied by that artery (ischemia and necrosis). Part of an arterial or venous thrombus may break off as an embolus, travel through the bloodstream, and become lodged elsewhere as an embolism. This type of embolism is called a thromboembolism. Complications can arise when a venous thromboembolism (commonly referred to as VTE) occurs in the lungs as a pulmonary embolism. An arterial embolism can travel further into the affected blood vessel, where it can become lodged as an embolism.

A distinction must be made between arterial thromboses caused by clot formation in arteries (e.g., myocardial infarction, stroke) and venous thrombotic events, such as phlebitis, venous thrombosis and pulmonary embolism.

In Germany, around 100,000 people (around 500.000 arcross Europe) die each year from venous thromboembolism due to thrombotic events.

Arterial thrombotic events such as myocardial infarction and stroke occur mostly in individuals with "classic" risk factors (hypertension, diabetes, lipid metabolism disorders, obesity, nicotine consumption) and are predominantly found in older age. If arterial thrombotic events occur in individuals without corresponding risk factors, it must be remembered that coagulation defects may also increase the risk of arterial thrombosis and contribute to the development of corresponding thrombotic events. Coagulation workup would be advised when arterial events occur in young patients without appropriate risk factors or without an adequate risk profile. Examples of coagulation defects with an increased risk of arterial thrombotic events would include antiphospholipid syndrome (APLS) and severe antithrombin deficiency.

Venous thrombotic events, particularly phlebitis, venous thrombosis, and pulmonary embolism, are more common in individuals who have a congenital or acquired hypercoagulability of the blood ("thrombophilia") during life. Common congenital risk factors in venous thrombotic events include factor V Leiden mutation, prothrombin mutation, and protein C, protein S, and antithrombin deficiency. An acquired disorder associated with, among other things, an increased risk of venous thrombotic events is the so-called antiphospholipid syndrome (APLS). However, numerous other factors are now known to lead to hypercoagulability of the blood or to indicate existing hypercoagulability, thus increasing the risk of clot formation in the venous system. It should be taken into account that hormonal contraceptives (the pill) and the use of hormone replacement therapy (HRT) increase the risk of thrombosis.

For both arterial and venous clot formation, "blood diluting agents" (antithrombotics) are administered for treatment. Depending on the type of clot formation, the type of defect present, and other factors, the treatment of different patients can vary considerably. In addition, the tolerability, efficacy and dosage of the drugs are not the same in all people, but can differ significantly.

Deep vein thrombosis (DVT) is the formation of a blood clot in a deep vein. It most commonly affects the veins of the leg, such as the femoral vein. Three factors are important for the formation of a blood clot in a deep vein: the speed of blood flow, the thickness of the blood, and the nature of the vessel wall. Classic signs of deep vein thrombosis include swelling, pain and redness of the affected area.

Paget-Schroetter disease or upper extremity DVT (UEDVT) is the blockage of a vein in the arm (e.g., axillary vein or subclavian vein) by a thrombus. The condition usually occurs after vigorous exercise and is most commonly seen in younger, otherwise healthy individuals. Men are more commonly affected than women.

Budd-Chiari syndrome is the occlusion of a hepatic vein or the hepatic portion of the inferior vena cava. This form of thrombosis is manifested by abdominal pain, ascites, and an enlarged liver. Treatment varies between therapy and surgery through the use of shunts.

Portal vein thrombosis affects the hepatic portal vein, which can lead to portal hypertension and a decrease in blood flow to the liver. Portal vein thrombosis usually occurs in association with another disease such as pancreatitis, cirrhosis, diverticulitis or cholangiocarcinoma.

Renal vein thrombosis is a blockage of the renal vein by a thrombus. This usually results in decreased outflow from the kidney.

Cerebral venous sinus thrombosis (CVST) is a rare form of stroke that results from blockage of the dural venous sinuses by a thrombus. Symptoms include headache, visual disturbances, symptoms of stroke such as weakness of the face and limbs on one side of the body, and seizures. Diagnosis is usually made by a CT scan or MRI. The majority of those affected make a full recovery. The mortality rate is 4.3%.

Jugular vein thrombosis can occur due to infection, intravenous drug use, or malignancy. Jugular vein thrombosis can result in a number of complications including: systemic sepsis, pulmonary embolism, and papilledema. Jugular vein thrombosis, while characterized by a sharp pain at the site of the vein, can be difficult to diagnose as it may occur incidentally.

Cavernous sinus thrombosis is a special form of cerebral sinus thrombosis in which there is thrombosis of the cavernous sinus of the basal cranial dura due to retrograde spread of infection and endothelial damage from the facial triangle of danger. The facial veins in this area anastomose with the superior and inferior ophthalmic veins of the orbit, which drain directly posteriorly into the cavernous sinus through the superior orbital fissure. Staphylococcal or streptococcal infections of the face, such as nasal or upper lip pustules, can thus spread directly into the cavernous sinus, causing stroke-like symptoms such as double vision and strabismus, and spread of infection to meningitis.

Arterial thrombosis is the formation of a thrombus in an artery. In most cases, arterial thrombosis follows the rupture of an atheroma (a fatty deposit in the blood vessel wall) and is therefore called atherothrombosis. An arterial embolism occurs when the clot travels downstream and can affect any organ.

An arterial occlusion can also result from an embolism of blood clots originating in the heart ("cardiogenic" emboli). The most common cause is atrial fibrillation, which causes blood stasis in the atria that facilitates thrombus formation, but blood clots can also form in the heart for other reasons, such as infective endocarditis.

A stroke is a rapid decline in brain function due to a disruption in the blood supply to the brain. This may be due to ischemia, thrombus, embolus (a stuck particle), or hemorrhage. In thrombotic stroke, a thrombus (blood clot) usually forms around atherosclerotic plaques. Because the blockage of the artery is gradual, the onset of symptomatic thrombotic strokes is slower. Thrombotic strokes can be divided into two categories: Large vessel disease and small vessel disease. The former involves vessels such as the internal carotids, vertebral arteries, and the Circle of Willis. The latter can affect smaller vessels such as the branches of the circle of Willis.

Myocardial infarction or heart attack, is caused by ischemia (interruption of blood flow), often due to blockage of a coronary artery by a thrombus. This restriction results in inadequate oxygen supply to the heart muscle, leading to tissue death (infarction). A lesion forms, which is the infarct. A heart attack can quickly become fatal if emergency medical treatment is not given promptly. If it is diagnosed within 12 hours of the initial attack, thrombolytic therapy is initiated.

An arterial thrombus or embolus can also form in the limbs, resulting in acute limb ischemia.

Further diseases comprise hematologic diseases, such as thrombotic thrombocytopenic purpura, and cardiovascular diseases, such as atherosclerosis, stroke, myocardial infarction, or acute or chronic pulmonary thromboembolism.

Symptoms of thrombosis, include, but not limited to, pain in one leg (usually the calf or inner thigh), swelling in the leg or arm, chest pain, numbness or weakness on one side of the body, sudden change in your mental state.

Symptoms of thromboembolism, include, but not limited to, swelling, pain, cyanosis, heaviness, and cramping in the affected extremity.

Symptoms of pneumonia, e.g. for CAP, HAP, COVID-19, include, but not limited to, cough, which may produce greenish, yellow or even bloody mucus, fever, sweating and shaking chills shortness of breath, rapid and shallow breathing, sharp or stabbing chest pain that gets worse when you breathe deeply or cough, loss of appetite, low energy, and fatigue, nausea and vomiting, especially in small children, and confusion.

Symptoms of sepsis, include, but not limited to, fast heart rate, hyperventilation, feeling dizzy or faint, change in mental state - such as confusion or disorientation, diarrhea, nausea and vomiting, slurred speech, severe muscle pain, severe breathlessness, less urine production than normal - for example, not urinating for a day.

### Atherosclerosis and arteriosclerosis

"Atherosclerosis" is a condition of the disease arteriosclerosis in which the wall of the artery develops abnormalities called lesions. These lesions can lead to narrowing due to the deposition of atheromatous plaques. There are usually no symptoms at the onset of the disease, but when they develop, symptoms generally begin in middle age. In severe cases, coronary artery disease, stroke, peripheral artery disease, or kidney problems may occur, depending on which arteries are affected.

Atherosclerosis can be asymptomatic for decades. Atherogenesis is the development process of atheromatous plaques and characterized by remodeling of the arteries leading to subendothelial accumulation of fatty substances called plaques. The buildup of an atheromatous plaque is a slow process that develops over a period of several years through a series of complex cellular processes within the arterial wall and in response to a variety of local circulating vascular factors. Progression of atherosclerosis is accompanied by morphological and pathophysiological changes, including replacement of reversible early lesions (fatty streaks) by fibrous plaques.

The arteriosclerosis disease is a disease with three stages, wherein stage early and the late stage are also thrombocyte mediated phase of arteriosclerosis disease.

An "early stage" or "first stage" of atherosclerosis is characterized by the accumulation of lowdensity lipoprotein droplets, particularly within macrophages, leading to the formation of foam cells. The formation of foam cells and subsequent accumulation are the origin of atheroma plaque development. The atheroma consists of a lipid core and a fibrous cap, which together alter the wall thickness of the artery. The fibrous cap may eventually rupture, leading to thrombosis and complete obstruction of the lumen.

Significant narrowing due to plaque rupture and clots also occurs in the peripheral arteries that supply blood to the legs, arms and pelvis. Symptoms of narrowing include numbness in the arms or legs and pain. Another important site for plaque formation is the renal arteries, which supply blood to the kidneys. The appearance and accumulation of plaque leads to decreased blood flow to the kidneys and chronic kidney disease, which, as in all other areas, is usually asymptomatic only in the late stages.

A "late stage" or "third stage" of atherosclerosis is by plaque rupture, including repeated, and healing responses, wherein thrombocytes are involved.

Atherosclerosis can cause arterial thrombosis and affects people whose arteries are clogged with fatty deposits. An exact cause of the condition is not known. Risk factors include abnormal cholesterol levels, elevated levels of inflammatory markers, high blood pressure, diabetes, smoking, obesity, family history and unhealthy diet. Plaque is made up of fat, cholesterol, calcium and other substances found in the blood. Narrowing of the arteries restricts the flow of oxygenated blood to parts of the body. Diagnosis is made on the basis of a physical examination, an electrocardiogram and an exercise test, among other tests.

Treatment for the identified condition may include medications to lower cholesterol such as statins, blood pressure medications, or medications to reduce blood clotting such as aspirin. Various procedures may also be performed, such as percutaneous coronary intervention, coronary artery bypass grafting, or carotid endarterectomy.

Atherosclerosis usually begins at a young age and worsens with age. Almost all people are affected to some degree by age 65. In industrialized countries, atherosclerosis is the leading cause of death and disability.

Atherosclerosis can be asymptomatic for decades because the arteries at all plaque sites enlarge, leaving blood flow unaffected. Even most plaque ruptures do not cause symptoms until an artery becomes sufficiently narrowed or occluded by a clot. Signs and symptoms do not occur until severe narrowing or occlusion impedes blood flow to various organs to the point of causing symptoms. In most cases, patients do not realize they have the disease until other cardiovascular conditions such as stroke or heart attack occur. However, these symptoms vary depending on which artery or organ is affected.

### Nucleic acid

The term "nucleic acid" as used herein refers to a polymer containing at least two deoxyribonucleotides or ribonucleotides in single- or double-stranded form, and includes DNA, RNA, and hybrids thereof. DNA may be in the form of, for example, antisense molecules, RNA-DNA duplexes, a PCR product, chimeric sequences, derivatives, and combinations of these groups. RNA can be in the form of small interfering RNA (siRNA), dicer substrate dsRNA, small hairpin RNA (shRNA), asymmetric interfering RNA (aiRNA), microRNA (miRNA), mRNA and combinations thereof. DNA and RNA can form DNA-RNA hybrids. As used herein, the terms "polynucleotide" or "nucleic acid molecule" refers to messenger RNA (mRNA), RNA, genomic RNA (gRNA), plus strand RNA (RNA(+)), minus strand RNA (RNA(-)), genomic DNA (gDNA), complementary DNA (cDNA) or recombinant DNA. Polynucleotides include single and double stranded polynucleotides. Preferably, polynucleotides of the invention include polynucleotides or variants having at least about 50%, 55%, 60%, 65%, 70%, 75%, 80%, 85%, 90%, 95%, 96%, 97%, 98%, 99% or 100%) sequence identity to any of the reference sequences described herein, typically where the variant maintains at least one biological activity of the reference sequence.

A "gene" refers to a DNA region that encodes a gene product including regions regulating the production of the gene product, regardless of whether these sequences are adjacent to coding and/or transcribed sequences. A gene comprises, but is not limited to, promoter sequences, terminators, translational regulatory sequences such as ribosome binding sites and internal ribosome entry sites, enhancers, silencers, insulators, boundary elements, replication origins, matrix attachment sites, and locus control regions.

"Gene expression" is the conversion of the information encoded by a gene into a gene product. A gene product can be the direct transcription product of a gene (e.g., mRNA, tRNA, rRNA, antisense RNA, ribozyme, structural RNA or any other type of RNA) or a protein produced by translation of a mRNA. Gene products also include RNAs modified by processes such as capping, polyadenylation, methylation, and editing, as well as proteins modified by processes such as methylation, acetylation, phosphorylation, ubiquitination, ADP-ribosylation, myristoylation, and glycosylation. Gene expression is regulated by "expression regulatory element" or "regulatory sequence".

An "expression regulatory element" or "regulatory sequence", as used herein, refer to a segment of a nucleic acid molecule that is capable of increasing or decreasing the expression of certain genes in a cell of an organism. Expression regulatory element are known by a skilled person and comprise, for example, promoter, enhancer, silencer, poly-adenylation signal, histone binding sequences, and CpG islands. "Increased expression" of a certain gene refers to a number of protein molecules expressed from said gene being higher in the cell as compared to without elevated gene expression. "Decreased expression" of a certain gene refers to a number of protein molecules expressed from said gene being lower in the cell as compared to without lowered gene expression.

### Sequence Variation:

"Sequence variant" or "gene mutation" "or "mutation", as used herein, refers to a change in the nucleotide sequence of the genome of an organism, a virus, mitochondrial DNA, and/or extrachromosomal DNA. Mutations result from errors during DNA or viral replication, mitosis, or meiosis, or from other types of DNA damage (e.g., pyrimidine dimers caused by ultraviolet radiation), which can then undergo error-prone repair (especially microhomology-mediated end joining), cause an error in other forms of repair, or cause an error during replication (translesion synthesis). Mutations can also result from insertion or deletion of DNA segments due to mobile genetic elements.

Mutations can result in detectable changes in the observable characteristics (phenotype) of an organism. Mutations in genes can also have no effect, alter the product of a gene, or prevent the gene from functioning properly or completely. They can also occur in non-genetic regions. "Pathogenic" sequence variants refer to disease causing gene mutations. A skilled person knows how to find and identify a sequence variant, in particular the pathogenic sequence variant.

### Polypeptides

"Peptide" "polypeptide", "polypeptide fragment" and "protein" are used interchangeably, unless specified to the contrary, and according to conventional meaning, i.e., as a sequence of amino acids. Polypeptides are not limited to a specific length, e.g., they may comprise a full length protein sequence or a fragment of a full length protein, and may include post-translational modifications of the polypeptide, for example, glycosylations, acetylations, phosphorylations and the like, as well as other modifications known in the art, both naturally occurring and non-naturally occurring.

### Treatment and therapeutic methods

As used herein "treatment" or "treating," includes any beneficial or desirable effect on the symptoms or pathology of a disease or pathological condition, and may include even minimal reductions in one or more measurable markers of the disease or condition being treated. Treatment can involve optionally either the reduction or amelioration of symptoms of the disease or condition, or the delaying of the progression of the disease or condition. "Treatment" does not necessarily indicate complete eradication or cure of the disease or condition, or associated symptoms thereof.

In the present invention "treatment" or "therapy" generally means to obtain a desired pharmacological effect and/or physiological effect. The effect may be prophylactic in view of completely or partially preventing a disease and/or a symptom, for example by reducing the risk of a subject having a disease or symptom or may be therapeutic in view of partially or completely curing a disease and/or adverse effect of the disease.

In the present invention, "therapy" includes arbitrary treatments of diseases or conditions in mammals, in particular, humans, for example, the following treatments (a) to (c): (a) Prevention of onset of a disease, condition or symptom in a patient; (b) Inhibition of a symptom of a condition, that is, prevention of progression of the symptom; (c) Amelioration of a symptom of a condition, that is, induction of regression of the disease or symptom.

As used herein, "prevent," and similar words such as "prevented," "preventing" or "prophylactic" etc., indicate an approach for preventing, inhibiting, or reducing the likelihood of the occurrence or recurrence of, a disease or condition. It also refers to delaying the onset or recurrence of a disease or condition or delaying the occurrence or recurrence of the symptoms of a disease or condition. As used herein, "prevention" and similar words also includes reducing the intensity, effect, symptoms and/or burden of a disease or condition prior to onset or recurrence of the disease or condition.

In some embodiments, a time interval between treatment or prevention comprises a period of several hours (2, 3, 4, 5, 6, 7, 8, 9, 10, 11 or 12) several days (2, 3, 4, 5, 6, or 7), several weeks (1, 2, 3, 4, 5, 6, 7, or 8), or months (2, 3, 4, 5, or 6), preferably 1 weeks to 3 months. In some embodiments, the time interval from one treatment or prevention to the next subsequent treatment can be the same, nearly the same or can change.

In some embodiments, the treatment comprises administration of CFTR modulator, a derivative or pharmaceutical acceptable salt thereof, one or two or three or four times daily for more than one day, more than one week, more than one month, more than one year, preferably 4 times daily, 1 month.

In one embodiment, a method of treating or preventing a thrombosis and/or embolism and related condition in a subject in need thereof comprises administering an effective amount, e.g., therapeutically effective amount of a composition comprising the CFTR modulator contemplated herein. The quantity and frequency of administration will be determined by such factors as the condition of the patient, and the type and severity of the patient's disease, although appropriate dosages may be determined by clinical trials.

The administration of the compositions contemplated herein may be carried out in any convenient manner, including by aerosol inhalation, injection, ingestion, transfusion, implantation or transplantation. In a preferred embodiment, compositions are administered orally in tablet form.

The treatment of the present invention may be employed or administered as deemed suitable by a skilled practitioner. For example, in some embodiments, a time interval between treatments comprises a period of several hours (2, 3, 4, 5, 6, 7, 8, 9, 10, 11 or 12) several days (2, 3, 4, 5, 6, or 7), several weeks (1, 2, 3, 4, 5, 6, 7, or 8), or months (2, 3, 4, 5, or 6), preferably 1 day. In some embodiments, the time interval from one treatment to the next subsequent treatment can be the same, nearly the same or can change. In some embodiments, the treatment period in which the CFTR modulator, preferably ivacaftor, are administered to the subject can be >1 day, >2 days, >3 days, >4 days, >5 days, >6 days, >7 days, >8 days, >9 days, >10 days, >11 days, >12 days, >13 days, >2 weeks, >3 weeks, >1 month, > 2 months, > 3months, > 4 months, > 6 months, preferably 10 weeks.

### Treatment of atherosclerosis disease and/or thrombosis

CFTR modulator administration is configured to reduce calcium influx in target cells, preferably in thrombocytes, to reduce pathological thrombocyte activation, adhesion and/or aggregation, and/or to reduce a level of thrombocyte activation to a level, or below a level, present prior to treatment.

Treatment criteria comprise a positively diagnosed disease selected from a group of pulmonary disorders, thrombocytopathy, coagulopathies, infectious diseases, CAP, HAP, COVID-19, systemic or extrapulmonary infectious, inflammatory diseases, rheumatic diseases, such as systemic lupus erythematosus or Schönlein-Henoch purpura, hematologic diseases, such as thrombotic thrombocytopenic purpura, cardiovascular diseases, such as arteriosclerosis, stroke, myocardial infarction, or acute or chronic pulmonary thromboembolism.

Treatment criteria comprise a positively detected pathological thrombocyte hyperactivation, hypercoagulative state, increased intracellular Ca2+ levels, and/or increased expression of activation marker at the surface of thrombocyte including CD62p, CD63, and/or CD41/CD61.

Treatment criteria comprise a positively detected or a risk of developing coagulopathies, thrombocytopathy, hypercoagulative state, embolism, thrombosis, thrombotic event, arteriosclerosis, thromboembolism, intravascular coagulation, thrombotic microangiopathy, pneumonia-associated hyperactivation of thrombocytes, and/or pathological thrombocyte hyperactivation.

### Immune cells and Inflammation

An "immune cell" includes any cell of the vertebrate immune system, including lymphocytes such as B-cells, cytotoxic T-cells (i.e., CD8+ T-cells), helper T-cells (i.e., CD4+ T-cells, including Th1 and Th2 cells), natural killer cells, and γδ T-cells, monocytes, macrophages, neutrophils, dendritic cells, mast cells, eosinophils, and basophils.

The term "Inflammation" also refers to the biological response of a tissue to noxious stimuli such as pathogens, damaged cells (e.g., wounds), and/or irritants. The term "inflammatory response" refers to a specific mechanism that causes and regulates inflammation and, in addition to other mechanisms known in the art, activation migration of immune cells, or cytokine production, vasodilatation. Ideally, inflammation is a protective function of the intended body that both eliminates harmful stimuli and initiates the process of healing one or more affected tissues. However, hyperinflammation or chronic inflammation, described herein, is associated with various diseases such as viral infections, e.g. SARS-CoV-2 infection, hay fever, atherosclerosis, rheumatoid arthritis and other diseases known in the medical community. Pathogenetically, a disturbed immune regulation with persistent immune activation is suspected. Hyperinflammation or chronic inflammation can result in platelet (hyper-) activation by means of constant exposure to inflammatory mediators

### Human pathogen and pathogenic infection

A "human pathogen" refers to any biological agent or noxious agent of a physical or chemical nature being capable of causing disease in humans.

A pathogenic infection can be a viral, bacterial, fungal or parasitic infection.

The human pathogen causing a viral infection can be selected from the group consisting of rhinoviruses, coronaviruses, such as SARS-CoV-1 or SARS-CoV-2, MERS, influenza virus, respiratory syncytial virus (RSV), adenovirus, parainfluenza, herpes simplex virus, or cytomegalovirus pneumonia, respiratory syndrome virus, dengue virus, avian flu virus, swine flu virus, ebola virus, yellow fever, and entero virus.

The human pathogen causing a bacterial infection can be selected from the group consisting of chlamydia, haemophilus influenza, staphylococcus aureus, pseudomonas species such as pseudomonas aeruginosa, klebsiella pneumoniae, mycoplasma pneumoniae, chlamydophila pneumoniae, legionella pneumophila, moraxella catarrhalis, streptococcus species such as streptococcus pneumoniae, streptococcus viridans, streptococcus faecalis, enterobacter, salmonella, escherichia coli, proteus, serratia, or neisseria meningitidis.

The human pathogen causing a fungal infection can be selected from the group consisting of aspergillus fumigatus, aspergillus flavus, candida infection (e.g., candida albicans), histoplasma capsulatum, blastomyces, cryptococcus neoformans, pneumocystis jiroveci, pneumocystis pneumoniae, or coccidioides immitis.

The human pathogen causing a parasite infection selected from the group consisting of malaria infection including plasmodium falciparum infection, plasmodium malariae infection, plasmodium ovale infection, and plasmodium vivax infection.

Pathological thrombocyte hyperactivation, aggregation, and/or hypercoaglupathy can occur in a pulmonary diseases, including ARDS, VILI, TRALI or pneumonia. A pneumonia, caused by a human pathogen, is a lung diseae comprising community-acquired pneumonia (CAP), hospital-acquired pneumonia (HAP), and severe community-acquired pneumonia (sCAP). sCAP is present when the inflammation localized in the lungs spreads generalized to the body and leads to sepsis-associated complications such as sepsis, septic shock or organ failure. Ventilator-associated pneumonia (VAP) is a special form of HAP. For CAP common pathogens are Streptococcus pneumoniae, influenza virus, Haemophilus influenzae, Mycoplasma pneumoniae, Chlamydophila pneumoniae and rarely Legionella pneumophila. For HAP common pathogens are Pseudomonas aeruginosa, Enterobacter, E. coli, Proteus, Serratia, Klebsiella pneumoniae and further multiresistant pathogens.

### Viral infection and viral load

As used herein, the term "viral infection" describes a disease state in which a virus invades healthy cells. Viruses use the cell's reproductive machinery to multiply or replicate and finally dissolve the cell, leading to cell death, the release of viral particles and infection of other cells by the newly produced progeny viruses. The term "viral load" refers to a quantitative measure of viral genomes per invaded cell. The determination of viral genomic material may be employed in such a method. A latent infection by certain viruses is also a possible consequence of a viral infection. The term "viral growth" relates to the infection and replication of a virus and the production of viral particles during and after the infection of a host cell.

### Detection of virus, bacteria and/or fungus

Infectious agents can be detected in suitable test samples. Examples of samples include swabs from wounds, stool samples, body fluids such as urine, cerebrospinal fluid, secretions (secretions) from the respiratory tract, and a special form of blood sample (blood cultures). A skilled person is capable of finding a method for detecting an infectious agent of interest without any effort. Typical methods detecting an infectious agent comprise PCR, quantitative PCR, ELISA, FACS, whole genome sequencing, whole transcriptome sequencing, microscopic examination of specimens with special stains, antigen detection, western blots, or microbiological differential smear. In one embodiment, an infectious agent can be detected in a sample obtained from a ME/CFS patient. A frequently recurrent HSV-1, HSV-2 or VZV infection can be diagnosed clinically. If there is a history of tick infections, a Borrelia ELISA and, if positive, a complementary Western blot should be performed. Suitable diagnostic approaches are known to one skilled in the art.

### Coagulopathies, thrombosis, and thromboembolic events associated with SARS corona virus infection

Coagulopathies, thrombosis, and thromboembolic events may occur in a human subject that has, had or is at risk of developing a severe acute respiratory syndrome (SARS) coronavirus infection.

Coronaviruses are a group of related viruses that cause diseases in mammals and birds. The scientific name for coronavirus is Orthocoronavirinae or Coronavirinae. Coronavirus belongs to the family of Coronaviridae. The family is divided into Coronavirinae and Torovirinae subfamilies, which are further divided into six genera: Alphacoronavirus, Betacoronavirus, Gammacoronavirus, Deltacoronavirus, Torovirus, and Bafinivirus. In humans, coronaviruses cause respiratory tract infections that can be mild, such as some cases of the common cold, and others that can be lethal, such as SARS, MERS, and COVID-19.

While the majority of cases result in mild symptoms, some progress to viral pneumonia, multi-organ failure, and thrombotic events.

A SARS coronavirus-2 (SARS-CoV-2) infection can cause pneumonia associated with increased risk of pulmonary vascular micro- and macrothrombosis, thrombotic microangiopathy, coagulopathy, thrombosis, embolism, and significant morbidity and lethality.

Various species of human coronaviruses are known, such as, without limitation, Human coronavirus OC43 (HCoV-OC43), of the genus β-CoV, Human coronavirus HKU1 (HCoV-HKU1), of the genus β-CoV, Human coronavirus 229E (HCoV-229E), α-CoV, Human coronavirus NL63 (HCoV-NL63), α-CoV, Middle East respiratory syndrome-related coronavirus (MERS-CoV), Severe acute respiratory syndrome coronavirus (SARS-CoV), Severe acute respiratory syndrome coronavirus 2 (SARS-CoV-2). Advances in nucleic acid sequencing technology (commonly termed Next-Generation Sequencing, NGS) are providing large sets of sequence data obtained from a variety of biological samples and allowing the characterization of both known and novel virus strains. Established methods are therefore available for determining a Coronavirus infection.

SARS is induced via droplet transmission and replication of the virus could be shown in the upper and lower respiratory tract or gastrointestinal mucosa. In parallel the virus is also capable of directly invading cells of different organs such as liver, kidney, heart and brain. Another distinct mechanism appears to be the direct invasion of the virus in T-cells. A significant number of SARS patients, who suffer COVID-19, have a clinically low concentration of lymphocytes and thrombocytes in the blood, also known as lymphocytopenia and thrombozytopenia, respectively. Clinically, patients show respiratory symptoms such as dry cough and shortness of breath, fever or diarrhea. But symptoms associated with acute liver injury, heart injury or kidney injury can also occur. In a less severe state of SARS, patients can show mild or even no symptoms.

Clinical and scientific investigations show that SARS-CoVs bind to the epithelial cells via the Angiotensin converting enzyme 2 receptor (ACE2). ACE2 is a cell membrane linked carboxypeptidase which is expressed in vascular endothelia, kidney, bladder, heart, nasal mucosa, bronchus and lung. As one consequence binding of the virus leads to an epithelial and endothelial cell damage along with vascular leakage, which triggers a secretion of pro-inflammatory cytokines and chemokines. The virus also mediates ACE2 downregulation and shedding which further promotes a dysfunctional renin-angiotensin system (RAS). Once the RAS is disturbed it can lead an inflammatory response and to vascular permeability. Focusing on the respiratory system, ACE2 shedding can lead to pulmonary vascular permeability and subsequently to pulmonary edema.

Older patients (above 60 years), patients with chronic diseases (e.g. cardiovascular diseases, diabetes, cancer, COPD) or immune compromised patients are considered to be at a higher risk to face a severe development of SARS. Smoking and obesity are also considered as risk factors. Embodiments of a SARS Coronavirus include, without limitation, any coronavirus that induces a SARS or SARS-similar pathology. Particular embodiments include, without limitation, the SARS Coronavirus (SARS-CoV-1), MERS-CoV, and the SARS-CoV-2, which causes COVID-19. The strain SARS-CoV-2 causes COVID-19, a disease which brought about the ongoing 2019-2020 coronavirus pandemic. The disease was first identified in December 2019 in Wuhan, the capital of China's Hubei province, and spread globally. Common symptoms include fever, cough, and shortness of breath. Other symptoms may include muscle pain, diarrhea, sore throat, loss of taste and/or smell, and abdominal pain.

### Subject

As used herein, the term "subject" refers to a mammal, such as humans, but can also be another animal, such as a domestic animal (e.g. a dog, cat or the like), a farm animal (e.g. a cow, sheep, pig, horse or the like) or a laboratory animal (e.g. a monkey, rat, mouse, rabbit, guinea pig or the like).

The term "patient" refers to a "subject" suffering from or suspected of suffering from thrombosis, thrombotic event, embolism, thromboembolism, coagulopathies, thrombocytopathy, hypercoagulative state, artheriosclerosis. The subject to be treated can additionally suffer from cystic fibrosis.

### Host cell and target cell

The term "host cell" and "target cell", as used herein, refers to a single cell or cell culture that may be or has been a recipient of at least one of the agents described herein, individually or in combination. Host cells include progeny of a single host cell, which progeny may not necessarily be completely identical (in morphology or overall DNA complement) to the original parent cell due to natural, random or intentional mutations and/or changes. In one embodiment, the host cell also refers to a cell into which an infectious agent (e.g. a virus) has invaded or is capable of invading.

### Pharmaceutically acceptable salts, pharmaceutical compositions and methods of administration

The present invention also relates to a pharmaceutical composition comprising the compounds described herein. The invention also relates to pharmaceutically acceptable salts of the compounds described herein, in addition to enantiomers and/or tautomers of the compounds described.

In some embodiments, the CFTR modulator may be provided as (i) the compound itself (e.g., as a free base); (ii) a pharmaceutically acceptable salt of the compound; or (iii) part of a pharmaceutical composition. In some embodiments of the above methods, uses and pharmaceutical compositions, the additional therapeutic means may be provided as (i) the compound itself (e.g. as a free base); (ii) a pharmaceutically acceptable salt of the compound; (iii) or as part of a pharmaceutical composition.

"Pharmaceutically acceptable salts" of the compounds described herein include those resulting from said compounds when mixed with inorganic or organic acids or bases. In some embodiments, the salts may be produced in situ during the final isolation and purification of the compounds. In other embodiments, the salts may be produced from the free form of the compound in a separate synthesis step. The preparation of the pharmaceutically acceptable salts described above and other typical pharmaceutically acceptable salts is described in more detail in Berg et ah, "Pharmaceutical Salts", J. Pharm. ScL, 1977:66:1-19, which is incorporated herein by reference in its entirety. The pharmaceutically acceptable salts of a CFTR modulator are those that can be used in medicine. However, salts that are not pharmaceutically acceptable may be useful in producing a CFTR modulator or their pharmaceutically acceptable salts.

For CFTR modulators, suitable "pharmaceutically acceptable salts" refer to salts produced from pharmaceutically acceptable non-toxic bases, including inorganic and organic bases. Salts derived from inorganic bases include aluminium, ammonium, calcium, copper, ferric iron, ferrous iron, lithium, magnesium, manganese, manganese (II), potassium, sodium, zinc and the like. Specific embodiments include ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines, arginine, betaine, caffeine, choline, N,N'-dibcnzylcthylcncdiaminc, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

The term "pharmaceutical composition" refers to a combination of the agent as described herein with a pharmaceutically acceptable carrier. The phrase "pharmaceutically-acceptable" refers to molecular entities and compositions that do not produce a severe allergic or similar untoward reaction when administered to a human. As used herein, "carrier" or "carrier substance" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Supplementary active ingredients can also be incorporated into the compositions.

The pharmaceutical composition containing the active ingredient may be in a form suitable for oral use, for example, as tablets, chewing tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions. Tablets contain the active ingredient in admixture with non-toxic pharmaceutically acceptable excipients which are suitable for the manufacture of tablets. The tablets may be uncoated or they may be coated by known techniques to delay disintegration and absorption in the gastrointestinal tract and thereby provide a sustained action over a longer period.

In one embodiment, the pharmaceutical composition comprising the CFTR modulator, preferably ivacaftor, described herein is supplied to the subject three times daily as tablet or liquid formulation, preferably 1-2 mg/kg/day CFTR modulator, preferably administered either as tablet or via a gastric tube.

The CFTR modulators are usually formulated into pharmaceutical compositions. The pharmaceutical compositions may optionally further comprise one or more additional pharmaceutically active compounds including one or more further CFTR modulators as defined above and/or a combination thereof. For example, ivacaftor may be combined with a CFTR corrector selected from Lumacaftor or Tezacaftor. The components for a kit of parts of the invention may be formulated for simultaneous administration or for administration in any sequence. The components may also be for repeated administration.

Administration of the compounds of the invention, or their pharmaceutically acceptable salts, in pure form or in an appropriate pharmaceutical composition, can be carried out via any of the accepted modes of administration or agents for serving similar utilities. Thus, administration can be, for example, orally, nasally, parenterally, topically, transdermally, or rectally, sublingually, intramuscular, subcutaneously, or intravenously in the form of solid, semi-solid, lyophilized powder, or liquid dosage forms, such as for example, tablets, suppositories, pills, soft elastic and hard gelatin capsules, powders, solutions, suspensions, or aerosols, or the like, preferably in unit dosage forms suitable for simple administration of precise dosages. The compositions will include a conventional pharmaceutical carrier or excipient and a compound of the invention as the/an active agent, and, in addition, may include other medicinal agents, pharmaceutical agents, carriers, adjuvants, etc.

Dosage levels of the order of from about 1 mg to about 5000 mg per day are useful in the treatment of the indicated conditions of human subject up to 30kg body weight. For example, thrombosis and embolism may be effectively treated by the administration of from about 2 mg to about 2000mg, preferably 20 mg to about 1000 mg, more preferably 100 mg to about 200 mg, of the CFTR modulator, per patient up to 30kg body weight per day.

Dosage levels of the order of from about 1 mg to about 5000 mg per day are useful in the treatment of the indicated conditions of human subject above 30kg body weight. For example, thrombosis and embolism may be effectively treated by the administration of from about 4 mg to about 4000mg, preferably 40 mg to about 2000 mg, more preferably 200 mg to about 400 mg, of the CFTR modulator, per patient above 30kg body weight per day.

The amount of active ingredient that may be combined with the carrier materials to produce a single dosage form will vary depending upon the host treated and the particular mode of administration. For example, a formulation intended for the oral administration of humans may vary from about 5 to about 95% of the total composition. Dosage unit forms will generally contain between from about 1 mg to about 500 mg of active ingredient. It will be understood, however, that the specific dose level for any particular patient will depend upon a variety of factors including the activity of the specific compound employed, the age, body weight, general health, sex, diet time of administration, route of administration, rate of excretion, drug combination and the severity of the particular disease undergoing therapy. The dosage effective amount of compounds according to the invention will vary depending upon factors including the particular compound, toxicity, and inhibitory activity, the condition treated, and whether the compound is administered alone or with other therapies.

### Therapeutically effective and therapeutically relevant

The phrase "therapeutically effective" is intended to include, within the scope of sound medical judgment, excessive toxicity, irritation, allergic reactions, and/or other problems or complications, but commensurate with a reasonable benefit/risk ratio. As used herein to refer to the compounds, compositions, combinations and/or dosage forms suitable for use in contact with a subject that produces a result that in and of itself helps to treat and/or cure a disease.

A "therapeutically relevant amount", "therapeutically relevant dosage" or "therapeutically effective amount" of an agent or therapeutic means, such as a CFTR modulator, i.e. ivacaftor, is an amount sufficient to produce the desired effect, e.g., improving CFTR chloride ion channel activity and chloride flow relative to the levels detected in the absence of a CFTR modulator. Improvement of a CFTR chloride ion channel activity and chloride flow is achieved when the level obtained with a CFTR modulator. relative to level prior treatment is about 95%, 90%, 85%, 80%, 75%, 70%, 65%, 60%, 55%, 50%, 45%, 40%, 35%, 30%, 25%, 20%, 15%, 10%, 5%, or 0%. Suitable assays for measuring chloride flow, intracellular chloride level, and/ or intracellular calcium levels include, for example, assaying chloride ions using techniques known to those skilled in the art, such as single channel activity by patch clamp technique, short circuit currents in Ussing chambers, or changes in intracellular Cl levels by CI- sensitive fluorescent dyes as a function of extracellular CI- concentrations and phenotypic assays known to those skilled in the art.

By "increase," "improving," "enhancing," "elevate" or "amend" of CFTR chloride ion channel activity and chloride flow by a CFTR modulator, i.e. ivacaftor, is meant as a detectable rise in CFTR chloride ion channel activity and chloride flow relative to a given CFTR modulator, i.e. ivacaftor. The extent of decrease in intracellular calcium levels, and increase of CFTR chloride ion channel activity and chloride flow, i.e. ivacaftor, may be determined relative to the intracellular chloride ion level without the presence of a CFTR modulator, i.e. ivacaftor, preferably before initiation of treatment with a CFTR modulator, i.e. ivacaftor. A detectable increase in intracellular calcium levels, CFTR chloride ion channel activity and chloride flow may be about 5%, 10%, 15%, 20%, 25%, 30%, 35%, 40%, 45%, 50%, 55%, 60%, 65%, 70%, 75%, 80%, 85%, 90%, 95%, or 100% than the intracellular calcium levels, CFTR chloride ion channel activity and chloride flow detected before initiation of treatment with CFTR modulator, i.e. ivacaftor. A decrease in thrombocyte hyperacitivation, hypercoagulative state, and intracellular calcium levels and improved CFTR chloride ion channel activity and chloride flow by the CFTR modulator is typically understood and measured by a decrease in intracellular calcium levels, and improved CFTR chloride ion channel activity by a responder cell in vitro or in vivo.

"Systemic administration," as used herein, refers to the administration of a composition that results in broad biodistribution of CFTR modulator, preferably ivacaftor, within an organism. Systemic administration means exposing a therapeutic amount of an agent to preferred parts of the body. Systemic administration of the composition may be accomplished by any means known in the art, e.g., intravenously, subcutaneously, intraperitoneally.

### Combined administration

According to the present invention, the term "combined administration", otherwise known as co-administration or joint treatment, encompasses in some embodiments the administration of separate formulations of the compounds described herein, whereby treatment may occur together, within minutes of each other, in the same hour, on the same day, in the same week or in the same month as one another. Alternating administration of two agents is considered as one embodiment of combined administration. Staggered administration is encompassed by the term combined administration, whereby one agent may be administered, followed by the later administration of a second agent, optionally followed by administration of the first agent, again, and so forth. Simultaneous administration of multiple agents is considered as one embodiment of combined administration. Simultaneous administration encompasses in some embodiments, for example the taking of multiple compositions comprising the multiple agents at the same time, e.g. orally by ingesting separate tablets simultaneously. A combination medicament, such as a single formulation comprising multiple agents disclosed herein, and optionally additional anti-viral and/or anti-inflammatory or autoantibody-targeting medicaments, may also be used in order to co-administer the various components in a single administration or dosage.

For example, one or more CFTR modulators can be administered to the human subject in combination. In another example, one or more CFTR modulators can be administered to the human subject in combination with at least non-CFTR modulator treatment.

A combined therapy or combined administration of one agent may occur together or precede or follow treatment with the other agent to be combined, by intervals ranging from minutes to weeks. In embodiments where the second agent and the first agent are administered separately, one would generally ensure that a significant period of time did not expire between the time of each delivery, such that the first and second agents would still be able to exert an advantageously combined synergistic effect on a treatment site. In such instances, it is contemplated that one would contact the subject with both modalities within about 12-24 h of each other and, more preferably, within about 3-12 h of each other, with a delay time of only about 6 h being most preferred. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

In the meaning of the invention, any form of administration of the multiple agents described herein is encompassed by combined administration, such that a beneficial additional therapeutic effect, preferably a synergistic effect, is achieved through the combined administration of the two agents.

### FIGURES

The invention is demonstrated by way of the example through the figures disclosed herein. The figures provided represent particular, non-limiting embodiments and are not intended to limit the scope of the invention.

### Short description of the figures:

**Figure 1****:** Platelets from COVID-19 patients are hyperactivated showing a significant reduction in [Ca2+]i response to platelet-activating agonists after pretreatment with ivacaftor.
**Figure 2****:** Pretreatment with the CFTR potentiator ivacaftor reduces agonist-induced platelet adhesion and aggregation in vitro.
**Figure 3****:** Retrospective risk analyses of the "Outcomes" of patients with CF in a large international cohort of CF patients from the TriNetX Real World depending on the use/non-use of one of the CFTR modulator ivacaftor, lumacaftor, tezacaftor or elexacaftor.

### Detailed description of the figures:

**Figure 1****: Platelets from COVID-19 patients are hyperactivated showing a significant reduction in [Ca2+]i response to platelet-activating agonists after pretreatment with ivacaftor. (A)** Overview of the COVID-19 patient and healthy donor study cohort classified according to the World Health Organization (WHO) clinical progression scale for COVID-1921. **(B)** Flow cytometry of surface expression of activation markers CD62p and CD63 on platelets from COVID-19 patients with moderate or severe disease course compared to healthy donors. **(C)** Surface expression of CD62p and CD63 on platelets isolated from blood of healthy donors and pre- treated with either vehicle, ivacaftor, or forskolin for 24h in vitro prior to agoniststimulation with 50 µM ADP, 50 µM TRAP6, or 5 µM PAF. **(D)** Representative trajectories of agonist- induced [Ca2+]i response monitored by loading platelets with the ratiometric Ca2+-sensitive dye Indo-1 AM and FACS after pretreatment with either vehicle, ivacaftor, or forskolin. **(E)** Quantification of [Ca2+]i transients measured in **(D)** as area-under-the-curve (AUC). Each circle **(B, C, E)** represents platelets from an individual patient/donor; bars indicate mean. *P < 0.05; **P < 0.005; ***P < 0.0005 (2-way ANOVA Holm-Šídák post hoc test **(B)** and with Geisser- Greenhouse correction **(C, E)).** Data are representative of **(B)** CD62p (n=20 (healthy), n=32 (moderate), n=22 (severe)) and CD63 (n=20 (healthy), n=32 (moderate), n=23 (severe)), **(C)** ADP, TRAP-6, or PAF following pretreatment with vehicle, ivacaftor, or forskolin (n=13 each for all samples), **(E)** ADP, TRAP-6, or PAF following pretreatment with vehicle, ivacaftor, or forskolin (n=8 each for "healthy" and "severe"). NIV, non-invasive ventilation; ECMO, extracorporeal membrane oxygenation.

**Figure 2****: Pretreatment with the CFTR potentiator ivacaftor reduces agonist-induced platelet adhesion and aggregation in vitro. (A)** Quantification of agonist-induced aggregation of platelets from patients with moderate or severe COVID-19 compared to healthy donors by multiple electrode aggregometry (MEA, Multiplate^{®} Analyzer, Roche Diagnostics International Ltd.). Blood samples were pretreated with either vehicle, ivacaftor, or forskolin for 15 min. prior to stimulation with ADP, TRAP6, or PAF. **(B)** Representative fluorescence microscopy images of platelet adhesion on collagen IV after 5 min of perfusion with re-calcified whole blood pretreated for 15 min with vehicle, ivacaftor, or forskolin and stimulated without or with the agonists ADP, TRAP-6, or PAF. Green signals represent CD42b+ platelets. **(C)** Quantification of the effect of ivacaftor on platelet adhesion (CD42b+ total area in field of view). **(D)** Retrospective risk analysis for fatal outcome in a large international cohort of CF patients with COVID-19 from the TriNetX Real World Database as a function of the use/non-use of any of the CFTR modulators ivacaftor, lumacaftor, or tezacaftor. Each circle **(A, C)** represents platelets from an individual patient/donor; bars indicate mean. *P < 0.05; **P < 0.005; ***P < 0.0005 (2-way ANOVA Holm-Šídák post hoc test **(A, C)).** Data are representative of **(A)** ADP, TRAP- 6, or PAF following pretreatment with vehicle, ivacaftor, or forskolin (n=7-19 for "healthy", n=8-15 for "moderate", and n=6-15 for "severe") and **(C)** ADP, TRAP-6, or PAF following pretreatment with vehicle, ivacaftor, or forskolin (n=4-8 for "healthy", n=9 for "moderate", and n=6-12 for "severe"). **(B)** Scale bars = 100 µm.

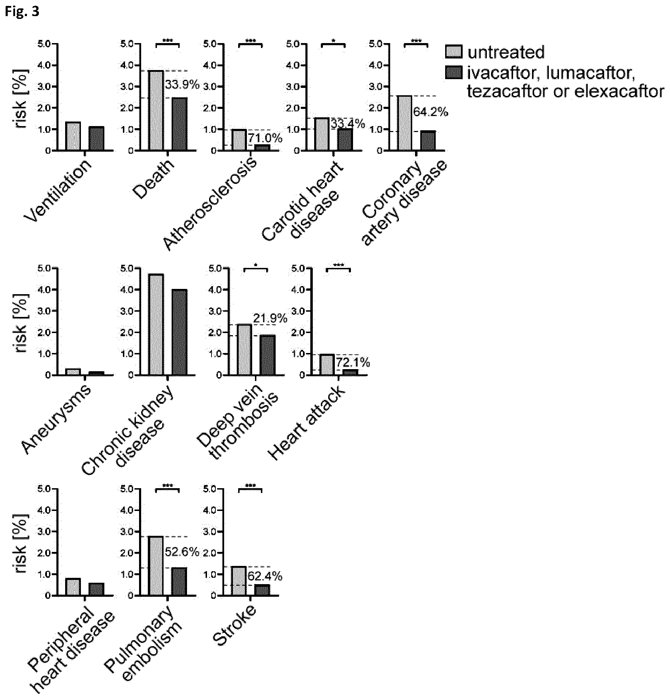

**Figure 3****: Retrospective risk analyses of the "Outcomes" of patients with CF in a large international cohort of CF patients from the TriNetX Real World depending on the use/non-use of one of the CFTR modulator ivacaftor, lumacaftor, tezacaftor or elexacaftor.** A total of 12,180 patients* with 6,081 patients* per group (-/+ ivacaftor, lumacaftor, tezacaftor, or elexacaftor therapy) were analyzed. *P < 0.05; **P < 0.005; ***P < 0.0005.

### EXAMPLES

The invention is demonstrated through the examples disclosed herein. The examples provided represent particular embodiments and are not intended to limit the scope of the invention. The examples are to be considered as providing a non-limiting illustration and technical support for carrying out the invention.

The examples below present the use of CFTR modulator in blood cells of COVID-19 patients and in CF patients, retrospectively. In preferred embodiments, platelet activation and aggregation is attenuated by administration CFTR modulator to blood cells of COVID-19 patients and healthy donors. In preferred embodiments, retrospective risk analysis for disease associated with thrombocyte hyperactivation, adhesion, and/or aggregation.

Described in more detail below is:
- Flow cytometry of thrombocyte activation and [Ca2+]i response in COVID-19 blood cells under CFTR treatement
- Electrode aggregometry and fluorescence microscopy for platelet adhesion and aggregation under CFTR treatement
- Retrospective risk analysis of Outcomes of CF patients being treated or not treatedthe CFTR potentiator ivacaftor reduces agonist-induced platelet adhesion and aggregation in vitro.

### Example 1: CFTR modulator attenuates platelet activation and aggregation in blood of COVID-19 patients and healthy donors

### Methods

Patient cohort: Citrated and hirudin blood from patients with moderate or severe COVID-19, or healthy donor controls was sampled at Charité - Universitätsmedizin Berlin, Germany (Pa-COVID-19 cohort study15,16 ethics approvals EA2/066/20 and EA2/075/15), and the Amsterdam University Medical Centers (AUMC), the Netherlands (ethics approval METc-number 2021.0520). Patients were included if they had a positive PCR test for SARS-CoV-2 and were over 18 years of age. Patients treated with anti-platelet drugs were excluded. Details on the study cohort are given in **Figure 1A****.**

**Flow Cytometry:** Blood samples were collected and stained as previously described3. In brief, blood was directly stained in dilution buffer (NaCl 137 mM, KCl 2.7 mM, MgCl2 1 mM, 4-(2-hydroxyethyl)-1- piperazineethanesulfonic acid (HEPES) 20 mM, glucose 5.6 mM, bovine serum albumin 1 g/l, pH 7.4.) containing mouse anti-human anti-CD42b (GPIb) Phycoerythrin (PE, clone HIP1), anti-CD62p (P-selectin) Phycoerythrin-Cyanine 7 (PE/Cy7, clone AK4), anti-CD63 (GP53) and Brilliant Violet 421 (clone H5C6), all from BioLegend (San Diego, CA, USA), before cells were fixed with 2 mL of 0.2 % PFA in PBS. In order to analyze isolated platelets, total citrate blood was centrifuged at 150g for 15 min to separate platelet rich plasma (PRP) from blood cells. PRP was collected and centrifuged again at 1000g for 15 min. Platelets were subsequently resuspended in 10% CPDA buffer in PBS (Sigma-Aldrich (St. Louis, MO, USA)) and incubated for 24h in the presence of either vehicle (DMSO, Sigma-Aldrich (St. Louis, MO, USA)), 10 µM ivacaftor (Cayman Chemicals, Ann Arbor, MI, USA)) or 5 µM forskolin (Fisher Scientific (Pittsburgh, PA, USA)) before staining was applied as described above. The analyses were performed on a BD FACSCanto^{™} II (BD Biosciences, Franklin Lakes, NJ, USA) flow cytometer equipped with violet (405nm), blue (488nm) and red (633nm) solid-state lasers. All measurements were analyzed with FlowJo (version 8, Tree Star, Inc., Ashland, OR, USA).

**Analysis of Ca2+ transients:** Platelet rich plasma (PRP) was stained with anti-CD42b, and loaded with Indo-1 AM (Invitrogen,Waltham, MA, USA)) for 30 min at 37 °C together in the presence of either vehicle (DMSO, 10 µM ivacaftor or 5 µM forskolin) under mild agitation. Stained PRP was diluted in Tyrode's Buffer (134 mM NaCl, 12 mM NaHCO3, 2.90 mM KCI, 0.34 mM Na2HPO4, 3 mM MgCI2, 2mM CaCI2, 20 mM HEPES, 5 mM Glucose, pH 7.4.) complemented with heparin and PGI1 before the fluorescence ratio of Ca2+-bound Indo-1 (405 nm) to Ca2+-unbound Indo-1 (530 nm) of CD42b+ platelets was monitored on a FACSymphony^{™} A5 (BD Biosciences, Franklin Lakes, NJ, USA) equipped with a ultraviolet (355 nm), violet (405 nm), blue (488 nm), yellow (561 nm), and red (637 nm) solid-state laser. After basal Ca2+ concentrations were monitored for 30 s, platelets were stimulated with either 5µM PAF (Tocris Bioscience (Bristol, England, GB)), 50µM ADP (Sigma-Aldrich, St. Louis, MO, USA) or 50µM TRAP-6 (purchased from Cayman Chemicals (Ann Arbor, MI, USA)) before changes in intracellular Ca2+ concentration ([Ca2+]i) were monitored for additional 2 min. Results are expressed in arbitrary units as areaunder the curve (AUC).

**Multiple Electrode Aggregometry:** Whole blood impedance measurement using Multiple Electrode Aggregometry (MEA) was performed on the Multiplate^{®} analyzer by Roche (Basel, Switzerland)). Hirudin whole blood samples were treated with either vehicle (DMSO), 10µM ivacaftor or 5µM forskolin for 15 min at 37°C. 300 µl of hirudin whole blood was diluted in 300µL 0.9% NaCl at 37°C in the test cells stimulated with either 5µM PAF, 6.5µM ADP or 33µM TRAP6 (Roche (Basel, Switzerland) according to the manufacturer's instruction. Results are expressed in arbitrary units as area under the curve (AUC).

**Vascular Injury Flow Assay:** Citrated blood was stained with anti-CD42b ((GPIb) Allophycocyanin (APC, clone REA 185, Miltenyi Biotec, Bergisch Gladbach, Germany)) and treated with either vehicle (DMSO), 10µM ivacaftor or 5 µM forskolin for 15 min at 37°C before the microfluidic flow assay was performed as previously described2. In brief, pretreated whole blood was diluted 1:4 with modified Tyrode's Buffer (134mM NaCl, 12mM NaHCO3, 2.90mM KCI, 0.34 mM Na2HPO4, 3mM MgCI2, 2.1 mM CaCI2, 20mM HEPES, 5mM Glucose, 1 g/l BSA, adjusted to a pH of 7.4.) immediately before perfusion with a flow rate of 20mL/h for 5min through collagen IV coated microchannel flow chambers (6 Channel µ-Slide VI 0.4, Ibidi, Martinsried, Germany). After perfusion, cells were fixed with 4% PFA for 30 min and washed thoroughly with PBS. Five randomly selected regions of interest (ROI) for each experimental condition were imaged with an Invitrogen^{™} EVOS^{™} fluorescent microscope and quantification of the total CD42b+ covered area was carried out with ImageJ (Version 13.0.6 by the US National Institutes of Health and the LOCI, University of Wisconsin).

**TriNetX Real World Database Analyses:** COVID-19-positive cystic fibrosis patients were identified via the ICD-10 code E84.9 and the presence of a SARS-CoV-2-related RNA diagnosis within the last 20 months. The data were collected from electronic health records in a TriNetX Real World database provided by a global health research network, with about 89 million patients from >60 healthcare organizations spanning 11 countries (https://trinetx.com/real-world-data/). This system deploys a linked and continually updated global health research network representing over 300 million patients. In the present work, we conducted a retrospective analysis of a large international COVID-19 cohort comprising 1.602 patients with ivacaftor, lumacaftor and tezacaftor and 103.065 Covid- 19-cases without *caftor therapy. The relation among the ivacaftor, lumacaftor and tezacaftor is 2:1:1. The group comparison was calculated for COVID-19-positive cystic fibrosis patients versus subjects without COVID-19. TriNetX analytics tools were used to obtain baseline characteristics, balance cohorts with propensity score matching, and analyze outcomes of interest in the final cohorts. The index event for each analysis was selected as the diagnosis of SARS-CoV-2 infection within the last 20 months. Baseline characteristics, including demographics, diagnoses, procedures, and medication, were obtained. Propensity score matching was used to balance cohorts. Propensity scores matched cohorts 1:1 using a nearest neighbor greedy matching algorithm with a caliper of 0.25 times the standard deviation. The primary outcome was defined as mechanical ventilation or death. Measures of association, including risk differences, risk ratios, and odds ratios, with their respective 95% CIs, were calculated using the TriNetX-tools.

### Results and Discussion

As compared to isolated platelets from healthy donors, platelets from COVID-19 patients showed enhanced surface expression of the activation markers CD62p (p-selectin) and CD63 as a function of disease severity **(****Figure 1B****).** This suggests a hypercoagulable state in COVID- 19. Increased expression of these activation markers could be recapitulated in platelets of healthy donors stimulated with either of the three platelet agonists adenosine diphosphate (ADP), thrombin receptor activating protein-6 (TRAP6), or platelet activating factor (PAF). Yet, upregulation of CD62p and CD63 was prevented in platelets pre-treated for 24 h with either the CFTR potentiator ivacaftor or forskolin, an adenylate cyclase activator and very effective, yet clinically not applicable inhibitor of platelet activation **(****Figure 1C****).** We next assessed the effect of ivacaftor on agonist-induced increases in intracellular Ca2+ concentration ([Ca2+]i) as the main "second messenger" of platelet activation. Activation with either ADP, TRAP6, or PAF induced a well-defined [Ca2+]i response that did not differ in shape or area- under-the-curve (AUC) between platelets of healthy donors or severe COVID-19 patients or healthy donors **(****Figure 1D and 1E****).** Except for TRAP6 stimulated healthy platelets, pretreatment with ivacaftor significantly attenuated the agonist-induced [Ca2+]i increase independent of patient status, qualitatively replicating again the effects of forskolin. Ca2+ influx mediates the shape change of activated platelets and their aggregation and adhesion. We consequently tested whether ivacaftor may also attenuate platelet aggregation in vitro. Multiple electrode aggregometry revealed that analogous to its effect on [Ca2+]i, ivacaftor pretreatment showed a general trend to reduce agonist-induced aggregation of platelets from both COVID- 19 patients and healthy donors, although this finding only reached significance for individual agonist-platelet combinations **(****Figure 2A****).** As such, ivacaftor again phenocopied the effect of forskolin. Finally, in a fluidic in vitro vascular disease model mimicking platelet-extracellular matrix interaction, ivacaftor again showed a general trend to reduce agonist-induced platelet adhesion in whole blood of COVID-19 patients with moderate or severe disease and healthy donors that reached significance for most agonist-platelet combinations and qualitatively mirrored the effects of forskolin **(****Figure 2B****,** C). As CFTR-modulators are clinically approved for CF, we retrospectively analyzed the TriNetX Real World database for CF-patients with COVID- 19 receiving ivacaftor, lumacaftor, or tezacaftor treatment in comparison to an untreated cohort. CFTR-modulator treatment substantially reduced the fatality risk by 55.9% (OR 0.438, CI 0.226-0.85; **Table 2** and **Figure 2D****)** while a 22.2% risk reduction for mechanical ventilation (OR 0.776, CI 0.432-1.394; **Table 2** and **Figure 2D****)** did not reach significance.

**Table 2: Statistical outcomes for cystic fibrosis patients with COVID-19: use/disuse of ivacaftor, lumacaftor or tezacaftor analysed for (A) Outcome: Mechanical ventilation, and (B) Outcome Death.**

| | | | | | | | |
|---|---|---|---|---|---|---|---|
| **(A)** | | | | | | | |

| **Drug compound** | **Number of patients in cohort** | **Outcome: Mechanical ventilation** | | | | | |
|---|---|---|---|---|---|---|---|
| | | Patients with outcome | Odds ratio | Confidence interval 95% | Risk (%) | Risk ratio | Confidence interval 95% |
| disuse | 5.162 | 58 | | | 1.124 | | |
| Use of Ivacaftor, Lumacaftor, or Tezacaftor | 1.602 | 14 | 0.776 | (0.432, 1.394) | 0.874 | 0.778 | (0.435, 1.39) |
| (B) | | | | | | | |

| **Drug compound** | **Number of patients in cohort** | **Outcome: Death** | | | | | |
|---|---|---|---|---|---|---|---|
| | | Patients with outcome | Odds ratio | Confidence interval 95% | Risk (%) | Risk ratio | Confidence interval 95% |
| disuse | 5.162 | 73 | | | | | |
| use of Ivacaftor, Lumacaftor, or Tezacaftor | 1.602 | 10 | 0.438 | (0.226, 0.85) | 0.624 | 0.441 | (0.228, 0.853) |

Here, we demonstrate that pharmacological modulation of CFTR by ivacaftor attenuates agonist-induced platelet activation, aggregation and adhesion. These protective properties were preserved in platelets of COVID-19 patients, pointing towards a therapeutic potential of CFTR- modulators in the therapy of severe COVID-19. This notion is supported by a retrospective analysis of multicentric patient data (TriNetX network), which showed better outcome in CF patients upon SARS-CoV-2 infection when on CFTR-modulator therapy. While the latter association may - in part - be confounded by differences in pre-COVID health status, CFTR- mutations, or health care availability, the notion of a protective effect of CFTR modulation is supported by the recently identified hypercoagulability of platelets with deficient or mutant CFTR. The protective effects of ivacaftor qualitatively resembled those of forskolin, an activator of adenylate cyclase commonly used in experimental studies to activate CFTR. While the non-specific effects of generalized adenylate cyclase activation prohibit clinical use of forskolin, CFTR-modulators have proven safe and effective in the treatment of CF patients and their use has not been associated with a higher risk of bleeding disorders; Conversely, CFTR- modulators seem beneficial in CF patients with catamenial hemoptysis. Previous work has linked loss of CFTR-function in platelets to increased activity of transient receptor potential canonical 6 (TRPC6), which mediates Ca2+ influx, activation and aggregation in platelets. Whether CFTR-modulators conversely reduce the open probability of TRPC6 or alternative Ca2+ influx channels, and if so, by which mechanism, remains to be elucidated. Since treatment of COVID-19 with aspirin has failed to show a significant reduction in thromboembolic events in the multicenter RECOVERY-trial a CFTR-modulating approach may serve as a more effective alternative in the clinical management of COVID-19 patients. That notwithstanding, our findings identify CFTR-modulators as potential therapeutic approach to prevent thromboembolic events in severe COVID-19, and other systemic disorders characterized by platelet hypercoagulability.

### Example 2: Risk-analyses for of CF-patients with and without receiving therapy using any "caftor"

A total of 12.180 patients including 6.081 patients per group were extracted from the TriNetX Real World Database and analysed whether **(Table 3**).This retrospective analysis of CF patients being treated with one or more of any "caftors" ("caftors" are in this example ivacaftor, lumacaftor, tezacaftor, elexacaftor) were compared to non-treated CF patients. CF patients treated with any "caftor" had a significantly reduced risk of developing coronary heart disease (63.7%) and atherosclerosis (73.9%). **(****Figure 3****)**

**Table 3: Statistical outcome for cystic fibrosis patients.**

| Outcome | Untreated group *no. of* | Treated group (ivacaftor, lumacaftor, tezacaftor or elexacaftor) *events* | Risk ratio (95% CI) | Risk difference (95% CI) | Odds ratio (95% CI) |
|---|---|---|---|---|---|
| Aneurysms | 20 | 10 | 0.5 (0.234, 1.067) | -0.164% (-0.341%, 0.012%) | 0.499 (0.233, 1.067) |
| Arteriosclerosis | 62 | 18 | 0.29 (0.172, 0.49) | -0.724% (-1.011%, -0.437%) | 0.288 (0.17, 0.488) |
| Carotid artery disease | 96 | 64 | 0.667 (0.487, 0.913) | -0.526% (-0.931%, -0,121%) | 0.663 (0.482, 0.912) |
| chron. ischem heart disease | 159 | 57 | 0.358 (0.265, 0.484) | -1.677% (-2.146%, -1.209%) | 0.352 (0.26, 0.478) |
| Chronic kidney disease | 289 | 245 | 0.848 (0.718, 1.001) | -0.724% (-1.452%, 0.005%) | 0.841 (0.707, 1.001) |
| Death | 230 | 152 | 0.661 (0.54, 0.809) | -1.283% (-1.902%, -0.663%) | 0.652 (0.53, 0.803) |
| Deep vein thrombosis | 146 | 114 | 0.781 (0.613, 0.0995) | -0.526% (-1.04%, -0.012%) | 0.777 (0.606, 0.995) |
| Diabetes | 979 | 1.695 | 1.731 (1.614, 1.857) | 11.774% (10.317%, 13.232%) | 2.014 (1.844, 2.2) |
| Heart attack | 61 | 17 | 0.279 (0.163, 0.476) | -0.724% (-1.007%, -0.44%) | 0.277 (0.161, 0.474) |
| Mechanical vertilation | 82 | 69 | 0.841 (0.612, 1.157) | -0.214% (-0.607%, 0.18%) | 0.84 (0.608, 1.159) |
| Peripheral artery disease | 51 | 37 | 0.725 (0.476, 1.106) | -0.23% (-0.531%, 0.071%) | 0.724(0.473, 1.107) |
| Pulmonary embolism | 171 | 81 | 0.474 (0.365, 0.616) | -1.48% (-1.986%, -0.974%) | 0.467 (0.357, 0.609) |
| Stroke | 85 | 32 | 0.376 (0.251, 0.564) | -0.872% (-1.218%, -0.525%) | 0.373 (0.248, 0.561) |

### References

1 Cui, S., Chen, S., Li, X., Liu, S. & Wang, F. Prevalence of venous thromboembolism in patients with severe novel coronavirus pneumonia. J Thromb Haemost 18, 1421-1424, doi:10.1111/jth.14830 (2020).
2 Klok, F. A. et al. Incidence of thrombotic complications in critically ill ICU patients with COVID-19. Thromb Res 191, 145-147, doi:10.1016/j.thromres.2020.04.013 (2020).
3 Leonard-Lorant, I. et al. Acute Pulmonary Embolism in Patients with COVID-19 at CT Angiography and Relationship to d-Dimer Levels. Radiology 296, E189-E191, doi:10.1148/radiol.2020201561 (2020).
4 Middeldorp, S. et al. Incidence of venous thromboembolism in hospitalized patients with COVID-19. J Thromb Haemost 18, 1995-2002, doi:10.1111/jth.14888 (2020).
5 Poissy, J. et al. Pulmonary Embolism in Patients With COVID-19: Awareness of an Increased Prevalence. Circulation 142, 184-186, doi:10.1161/CIRCULATIONAHA.120.047430 (2020).
6 Corrigan, D., Prucnal, C. & Kabrhel, C. Pulmonary embolism: the diagnosis, riskstratification, treatment and disposition of emergency department patients. Clin Exp Emerg Med 3, 117-125, doi:10.15441/ceem.16.146 (2016).
7 Lim, W. et al. Failure of anticoagulant thromboprophylaxis: risk factors in medicalsurgical critically ill patients*. Crit Care Med 43, 401-410, doi:10.1097/CCM.0000000000000713 (2015).
8 Ackermann, M. et al. Pulmonary Vascular Endothelialitis, Thrombosis, and Angiogenesis in Covid-19. N Engl J Med 383, 120-128, doi:10.1056/NEJMoa2015432 (2020).
9 Lang, M. et al. Hypoxaemia related to COVID-19: vascular and perfusion abnormalities on dual-energy CT. Lancet Infect Dis 20, 1365-1366, doi:10.1016/S1473-3099(20)30367-4 (2020).
10 Bernard, G. R. et al. Efficacy and safety of recombinant human activated protein C for severe sepsis. N Engl J Med 344, 699-709, doi:10.1056/NEJM200103083441001 (2001).
11 Nadel, S. et al. Drotrecogin alfa (activated) in children with severe sepsis: a multicentre phase III randomised controlled trial. Lancet 369, 836-843, doi:10.1016/S0140-6736(07)60411-5 (2007).
12 Abraham, E. et al. Drotrecogin alfa (activated) for adults with severe sepsis and a low risk of death. N Engl J Med 353, 1332-1341, doi:10.1056/NEJMoa050935 (2005).

## Claims

1. A cystic fibrosis transmembrane conductance regulator (CFTR) modulator for use in the treatment and/or prevention of a medical condition comprising thrombosis in a human subject.

2. The CFTR modulator for use according to the preceding claim, wherein the medical condition comprises an embolism and/or a thrombocyte-mediated coagulopathy, such as a hypercoagulability/thrombophilia.

3. The CFTR modulator for use according to any of the preceding claims, wherein the subject of treatment has suffered a thrombotic- and/or thromboembolic event.

4. The CFTR modulator for use according to any of the preceding claims, wherein the medical condition is associated with a human pathogenic infection, such as a viral, bacterial, fungal or parasitic infection.

5. The CFTR modulator for use according to the preceding claim, wherein the infection is a respiratory viral infection, preferably a SARS coronavirus infection, more preferably a SARS-CoV-2 infection.

6. A cystic fibrosis transmembrane conductance regulator (CFTR) modulator for use in the treatment and/or prevention of a medical condition comprising atherosclerosis and pathological thrombocyte activation.

7. The CFTR modulator for use according to the preceding claim, wherein the medical condition is selected from the group consisting of coronary artery disease, myocardial infarction, angina, stroke, transient ischemic attacks and peripheral artery disease.

8. The CFTR modulator for use according to any of the preceding claims, wherein the medical condition comprises a thrombocyte-mediated vascular disease.

9. The CFTR modulator for use according to any of the preceding claims, wherein the medical condition comprises pathological thrombocyte activation, adhesion and/or aggregation.

10. The CFTR modulator for use according to any of the preceding claims, wherein the CFTR modulator is administered to a non-cystic fibrosis subject.

11. The CFTR modulator for use according to any of claims 1 to 9, wherein the CFTR modulator is administered to a subject suffering additionally from cystic fibrosis.

12. The CFTR modulator for use according to any of the preceding claims, wherein the CFTR modulator is a CFTR activator, CFTR potentiator, CFTR corrector or CTFR amplifier, preferably a CFTR potentiator or a CFTR corrector.

13. The CFTR modulator for use according to any of the preceding claims, wherein the CFTR modulator is selected from the group consisting of ivacaftor, lumacaftor, tezacaftor and elexacaftor.

14. The CFTR modulator for use according to the preceding claim, wherein said CFTR modulator is ivacaftor.

15. The CFTR modulator for use according to any of the preceding claims, comprising the treatment of a medical condition comprising thrombosis in a subject suffering from a SARS coronavirus infection, preferably a SARS-CoV-2 infection, wherein the CFTR modulator is ivacaftor.

16. The CFTR modulator for use according to any of the preceding claims, wherein a dosage and frequency of said CFTR modulator, preferably ivacaftor, comprises:
(a) A dosage of 2-2000 mg/day, preferably at 20-1000 mg/day, more preferably at 100-200 mg/day to a human subject up to 30kg body weight, and 200-400 mg/day to a human subject above 30kg body weight, and/or
(b) A frequency of 2 to 4 times per day, preferably 2 times daily, wherein the route of administration is subcutaneously, intravenously or orally, preferably orally, via a tablet of 1 mg to 1000 mg per dose, more preferably 10 mg to 500 mg per dose, even more preferably 50 mg to 100 mg per dose to a human subject up to 30 kg body weight, and 100 mg to 200 mg per dose to a human subject above 30 kg body weight.

17. The CFTR modulator for use according to any of the preceding claims, wherein the treatment, dosage and/or frequency of CFTR modulator administration is configured to reduce calcium influx in target cells, preferably in thrombocytes, to reduce pathological thrombocyte activation, adhesion and/or aggregation, and/or to reduce a level of thrombocyte activation to a level, or below a level, present prior to treatment.

18. A pharmaceutical composition comprising a CFTR modulator for use in the treatment and/or prevention of a medical condition according to any of the preceding claims, wherein said CFTR modulator is in admixture with a pharmaceutically acceptable carrier and/or formulated in a pharmaceutically buffered solution.
